# EUROPEAN PATENT SPECIFICATION

(11) **EP 2 914 349 B1**
(45) Date of publication and mention of the grant of the patent: **19.12.2018**
(21) Application number: 13820912.7
(22) Date of filing: 28.10.2013
(51) Int. Cl.: A61Q 19/08, A61K 8/97, A61Q 19/02, A61Q 17/04

(54) **COMBINATION OF PLANT EXTRACTS, COSMETIC INGREDIENT AND COMPOSITION CONTAINING IT AND TOPICAL COSMETIC USE THEREOF**
KOMBINATION AUS PFLANZENEXTRAKTEN, EINEM KOSMETISCHEN INHALTSSTOFF UND EINER ZUSAMMENSETZUNG DAMIT SOWIE TOPISCHE KOSMETISCHE VERWENDUNG DAVON
COMBINAISON D'EXTRAITS DE PLANTE, INGRÉDIENT COSMÉTIQUE ET COMPOSITION LE CONTENANT ET SON UTILISATION COSMÉTIQUE TOPIQUE

(30) Priority: 30.10.2012 FR 1260377
(43) Date of publication of application: 09.09.2015
(73) Proprietor: Sederma, 78610 Le Perray en Yvelines (FR)
(72) Inventor: GRIZAUD, Claire-Marie, Houston, TX, 77088 (US); MONDON, Philippe, F-92120 Montrouge (FR)
(86) International application number: PCT/IB2013/059719
(87) International publication number: WO 2014/068470

(56) References cited:
- EP-A1- 1 462 110
- EP-A1- 1 870 094
- WO-A2-2008/028097
- US-A1- 2007 122 501
- DATABASE WPI Week 201232 Thomson Scientific, London, GB; AN 2012-A60101 XP002705288, & CN 102 283 406 A (COSMETIC CHEM WUHAN RES INST) 21 December 2011 (2011-12-21)
- DATABASE MEDLINE [Online] US NATIONAL LIBRARY OF MEDICINE (NLM), BETHESDA, MD, US; April 2007 (2007-04), CHANG YUN-HEE ET AL: "Inhibition of melanogenesis by selina-4(14),7(11)-dien-8-one isolated from Atractylodis Rhizoma Alba.", XP002705560, Database accession no. NLM17409509 & CHANG YUN-HEE ET AL: "Inhibition of melanogenesis by selina-4(14),7(11)-dien-8-one isolated from Atractylodis Rhizoma Alba.", BIOLOGICAL & PHARMACEUTICAL BULLETIN APR 2007, vol. 30, no. 4, April 2007 (2007-04), pages 719-723, ISSN: 0918-6158
- HSU M F ET AL: "Stimulating effects of Bacillus subtilis natto-fermented Radix astragali on hyaluronic acid production in human skin cells", JOURNAL OF ETHNOPHARMACOLOGY, ELSEVIER SCIENTIFIC PUBLISHERS LTD, IE, vol. 125, no. 3, 25 September 2009 (2009-09-25), pages 474-481, XP026586777, ISSN: 0378-8741, DOI: 10.1016/J.JEP.2009.07.011 [retrieved on 2009-07-18]
- DATABASE WPI Week 200738 Thomson Scientific, London, GB; AN 2007-400961 XP002705289, & KR 2006 0027764 A (AMOREPACIFIC CORP) 28 March 2006 (2006-03-28)

## Description

### TECHNICAL FIELD

The invention relates to a new cosmetic active ingredient, a topical composition containing it, and the topical use of said active ingredient and said composition to improve the appearance and condition of the skin, mucous membranes or skin appendages, and in particular for the treatment and/or prevention of signs of aging of skin and its appendages. The invention further relates to processes to enhance skin barrier effect, dermis and dermal-epidermal junction (DEJ), to protect against oxidation and pollutions, and to modulate pigment functions.

More generally, the present invention relates to the cosmetics, pharmaceutical and cosmeceutical industries that manufacture and/or use products to improve the appearance and general condition, to prevent and/or treat disorders and diseases of the skin, scalp, mucous membranes and skin appendages (such as body hair, eyelashes, eyebrows, nails, hair) of mammals, animals or humans.

### BACKGROUND ART

The demand for new products from these industries is increasing, particularly for new active ingredients that are derived from plants because they can combine efficiency, limiting the risk of irritation and allergy, lowering side effects and biodegradability, with the possibilities of labelling/certification and adequacy for a sustainable development and/or fair trade logic.

The face is emblematic of beauty, so it seems logical that it is a focus of attention: it can be displayed as is, be embellished or be hidden, to better communicate with or even attract someone else. Nevertheless, the face represents the part of the body that is most exposed to external aggressions which can affect its appearance. The sun, harsh weather, increased urban pollution and cigarette smoke gradually change the delicate balance that controls the skin's metabolism. The effects of these elements are evidenced by ungainly wrinkles and an uneven complexion. These phenomena are also amplified with age.

The skin consists of two compartments, one superficial, the epidermis, and the other deeper, the dermis, which interact.

Natural human epidermis is composed mainly of three types of cells which are keratinocytes, the great majority, melanocytes and Langerhans cells. Each of these cell types contributes with its own functions to the essential role played in the body by the skin, including the role of protecting the body from external aggression called "barrier function".

Epidermis is commonly divided into several layers, the deeper being a basal keratinocyte layer constituting the germinative layer of the epidermis, and the outermost being the horny layer (or *stratum corneum*) consisting of a plurality of keratinocyte layers at the terminal stage of differentiation and called corneocytes. Corneocytes are anucleate cells consisting mainly of a fibrous material containing cytokeratins, surrounded by a cornified envelope. Between the corneocytes, is a lipid-rich intercellular area, particularly lipids of the ceramide family. Also located in the *stratum corneum,* there are "natural moisturizing factors" (NMF - substances capable of binding water in the *stratum corneum,* which come from the sweat and sebum (urea, for example), and also from the process of cornification (pyrrolidone carboxylic acid, for example)) which maintain the level of skin moisture at an optimum level. These factors are soluble molecules and represent between 10 and 30% of the corneocyte content. The measurement of transepidermal water loss (TEWL) is a noninvasive method for assessing skin barrier function of the *stratum corneum.* It characterizes the evaporation of water contained in the skin: the more the skin barrier is damaged, the more water evaporates easily.

Dermis provides the epidermis with a solid support. It is also its nourishing element. It consists mainly of fibroblasts and an extracellular matrix composed mainly of collagen, elastin and a substance called the fundamental substance. These components are synthesized by fibroblasts. The cohesion between the epidermis and dermis is provided by the dermo-epidermal junction (DEJ).

The skin of different ethnic groups is not completely equivalent.

Different ethnic groups have different skin types. Besides color, there are less visible physiological differences; for example a number of studies show that Asian skins have certain special characteristics at the *stratum corneum* level.

It is known that darker skins are better protected against mechanical stresses. This is because the *stratum corneum* of darker skins is thicker and more cohesive. In contrast, light-skinned Asians have a thinner *stratum corneum*; this seems to have resulted in a greater sensitivity to external environmental stress as compared to other population groups. Although corneocyte size does not vary between different ethnic groups, the tested Asian populations have a higher number of ceramides than in other tested groups. However, ceramide levels systematically decline with age. Moreover, it has been proven that less adhesive is needed to alter the delicate transepidermal water loss (TEWL) balance in Asian groups than in other groups. Asian skin is therefore less resistant to mechanical stress. Their more abundant lipids stabilize but not enough offset the effects of having corneocytes that are fewer in number and less cohesive. It has been shown that young people of different ethnic backgrounds had no difference in hydration levels, but that with age, Asians and Caucasians had dryer skin in areas exposed to the sun, extreme weather and pollution than other ethnic groups. This may be related to less NMF quantity (Natural moisturizing factors; natural hydration). Subsequently, in Asians, it would seem that improving *stratum corneum* quality (more cells associated with maintaining or reinforcing intercorneocyte lipids) would solve the aforementioned problems.

Strengthening the epidermis would also moderate the sensitivity of skin to irritants, pollution and oxidants. To this end, we know that exposing skin to oxidants causes changes in the lipids of cell membranes and that this may produce overly active defense mechanisms and make the skin more sensitive. This is what creates "underlying" skin redness.

The Asian dermis seems to be the richer in collagen-I, but it also suffers from the effects of time. Maintaining synthesis of collagen I, the mainstay of the uniformity of the dermis, must be seek to effectively support epidermis, keeping it smooth, without unsightly bumps.

Furthermore, the dermal-epidermal junction also suffers from the effects of time. The role of this complex structure, in which coexist many molecules such as collagen IV and laminins, is pivotal because it enables the dermis to supply the epidermis with essential elements. The activation of dermal-epidermal junction synthesis is a good age-fighting indicator.

Finally, although Asian skins tend to wrinkle later then Western skins, they are prematurely marked by irregular pigmentation, leading to unevenness and discoloration. The clear, porcelain-like skin that is coveted as a symbol of femininity is tarnished by these visible chromophores. Therefore, it is important to moderate these defects.

The aim of the present invention is to meet these expectations, and proposes an active ingredient to reinforce skin barrier with regard to external stresses, to fight against the formation of wrinkles and to re-unify the complexion by reducing spots to make the skin more pleasant to watch.

CN20101203566 discloses a beverage for the treatment of freckles, said beverage comprising Chinese herbal medicine ingredients comprising *Bupleuri radix* and *Atractylodes macrocephala.*

EP1462110 discloses a medicament for the treatment of climacteric syndrome of women which is prepared from 18 ingredients among which are *Astragalus membranaceus* (Radix Astragali seu Hedysari), *Actractylodes macrocephalae* (Rhizoma Actractylodis Alba) and *Bupleurum falcatum* (Radix Bupleuri).

US2007/0122501 discloses formulations containing plant extracts in particular an *Astragalus membranaceus* extract, and their use in inducing telomerase activity in cells and to protect the cells from UV-induced apoptosis.

The scientific publication "Stimulating effects of Bacillus subtilis natto-fermented Radix astragali on hyaluronic acid production in human skin cells", Mei-Fang Hsu and al., Journal of Ethnopharmacology, 125 (2009) 474-481, discloses the stimulating effects of fermented preparations (HQB) of *Astragalus membranaceus* (Radix astragali) on the synthesis of hyaluronic acid and suggests that HQB may play a role in anti-ageing cosmetic applications.

WO2008/028097 discloses a method for skin care comprising administering the water insoluble fraction of *Astragalus membranaceus* for improving skin texture, reducing wrinkles, ultraviolet protection and anti-aging effects.

The scientific publication "Inhibition of melanogenesis by selina-4(14), 7(11)-dien-8-one isolated from Atractylodis Rhizoma Alba.", Chang Yun-Hee et al., Biological & Pharmaceutical Bulletin Apr 2007, vol.30, no. 4, April 2007 (2007-04), pages 719-723, discloses the inhibition of melanogenesis of a sesquiterpenoid compound, the selina-4(14), 7(11)-dien-8-one, isolated from *Atractylodes macrocephale* (Atractylodis Rhizoma Alba.).

EP1870094 discloses an anti-wrinkle composition comprising *Actractylodes macrocephale.* KR100072725B discloses compositions containing an extract of *Bupeurum falcatum* L. as an active ingredient to promote the growth of keratinocytes, remove reactive oxygen species produced by UV, promote biosynthesis of type 1 procollagen, and prevent skin aging.

### SUMMARY OF THE INVENTION

Unexpectedly, it was found in the context of the present invention, that a combination of extracts of plants known per se, is able to stimulate synergistically keratinocyte differentiation, laminin and hyaluronic acid synthesis by normal human keratinocytes and collagen synthesis, and is able to protect against lipid peroxidation and to decrease melanin synthesis.

Therefore, this combination is particularly useful to enhance the skin barrier effect, dermis and DEJ, to protect against oxidation and pollutions, and to modulate pigment functions and therefore to improve the appearance and general condition of the skin and its annexes, in particular for the treatment and/or prevention of skin aging signs that are associated to.

The cosmetic active ingredient of the present invention is consisting of the combination of extracts of three plants: *Astragalus membranaceus,* also known as Astragale or *Huang Qi,* which is a fabaceae from northern China and Mongolia, *Bupleurum falcatum* or *Bupleurum chinensis* or *Chai hu,* which is an apiaceae, and *Atractylodes macrocephalae* or *Atractyloides macrocephalae* or Bai Zhu, of the *Asteraceae* family.

Therefore a first object of the invention is a cosmetic active ingredient essentially consisting of (i) an extract of *Astragalus membranaceus,* (ii) an extract of *Bupleurum falcatum,* and (iii) an extract of *Atractylodes macrocephalae.*

"Plant extract" means according to the invention any form including, without limitation, the whole plant, a dried plant, a crushed or chopped plant, part or parts thereof, including without limitation, seeds, needles, leaves, roots or rhizomes, bark, cones, stems, rhizomes, dedifferentiated cells from callus, protoplasts, organs and organ systems, meristems, or components and/or constituents found in, or isolated from this plant, and/or portions of the plant, or extracts that are derived directly or synthetically from the plant, or any combination thereof. It must be understood that the "plant extract" also includes an ingredient, component, pure or semi-pure constituent, extract or solid or liquid derivative, of the plant or of the plant extract.

"Plant extract" means according to the invention a plant material that can be obtained either by usual techniques of extraction or by *in vitro* plant culture.

As examples, the usual extraction techniques that can be used include maceration, simple decoction, lixiviation, extraction under reflux, extraction by supercritical fluid extraction using ultrasound or microwaves, and counter flow extraction techniques, this list being not limited. The extraction solvents may be selected from water, propylene glycol, butylene glycol, glycerin, caprylic/capric PEG-6 glycerides, polyethylene glycol, methyl ethers and/or ethyl diglycols, cyclic polyols , ethoxylated or propoxylated diglycols, alcohols (methanol, ethanol, propanol, butanol, isobutanol and isopropanol), ketones (in particular acetone), or any mixture of these solvents.

According to a first mode, the extract can be obtained by maceration of the part of the plant in water, or in a solvent consisting of a mixture of water and an organic solvent for example water-alcohol, or water-acetone, or water-propylene glycol, or water-butylene glycol, or in a pure solvent such as an alcohol and particularly ethanol. The plant part may be fresh or dry, and the plant/solvent ratio can vary for example and without limitation from 1/4 to 1/20. Advantageously, the extract preparation begins with the grinding of parts of the plant followed by maceration in the extraction solvent for several hours. The extraction can be carried out with stirring to improve performance. The extraction may be performed at room temperature or by increasing the temperature for example up to 50°C or 60°C. Once the extraction is completed, the solution is filtered. The solution thus obtained can be concentrated by any method known to one skilled in the art. Similarly, the solution can be lyophilized by any conventional method of freeze drying to thus obtain a powder. Alternatively, the filtrate is taken up in a mixture of solvents, for example a mixture of glycerin/water in a ratio ranging from 90/10 to 10/90, preferably 50/50 v/v. After a homogenization step followed by a final filtration step, the extract is ready for use. The homogenization can be performed at room temperature or by increasing the temperature for example up to 40°C or 50°C.

In another alternative, the extracts of each plant can be made separately, and the three extracts thus obtained can be mixed up to 20 to 80% to any hydrophilic matrix such as a gel, an aqueous buffer, glycerin or other physiologically acceptable short chain polyol. For example, 20% of each extract may be present and 40% glycerin is added to form the active ingredient of combination of plant extracts. According to a second mode, the invention covers an extract obtained by *in vitro* plant culture. Various techniques exist including culture of de- or in-differentiated cells, organ or tissue culture, or or *in vitro* micropropagation through somatic embryogenesis or vegetative multiplication. These cultures can be obtained from cell or tissue lines from different organs of the plant.

Obtaining an extract from plant cell culture has advantages with regard to the agro-industrial way (growing plants in the open fields and subsequent factory extraction): materials obtained are free of toxic substances (herbicides etc.), reproducibility is improved, biodiversity is preserved, etc.

All parts of the plant, including aerial parts, such as leaves, flowers or seeds or roots can be used. Preferably according to the invention, the extracts are extracts of roots or rhizomes of plants, as disclosed thereafter.

The active ingredient of the invention, a combination of extracts of three plants: *Astragalus membranaceus, Bupleurum falcatum,* and *Atractylodes macrocephalae,* can be obtained by combining the extracts of a plant with the two others, combining the three plant extracts, or by extraction of the three plants simultaneously or successively. Preferably, the three plants will be extracted together. The three plants or plant extracts can be combined in any proportions. The ratio between the plants (m/m) or extract (v/v) are preferably in a range between 5:1 and 1:5. Preferably according to the invention, the three plants or plant extracts are present in the same proportion in the combination (1:1:1).

Indeed it has now been found that such actives, which separately have little or no effect on keratinocyte differentiation, laminin and hyaluronic acid synthesis by keratinocytes, and collagen synthesis, protection against lipid peroxidation and decrease of melanin synthesis are able to when combined, and thus reinforce the skin barrier effect, dermis and DEJ, protect against oxidation and pollution, and modulate pigment functions.

The combination therefore has a synergistic effect. According to the invention, the effect shown by the combination is qualified as synergistic since the combination is superior to the simple superposition of the respective effects of each of its constituents.

Advantageously, the combination of extracts according to the invention can be used pure or diluted in a physiologically acceptable carrier or matrix, including a glycerin matrix.

The active ingredient according to the invention is for cosmetics, pharmaceutical or cosmeceutical, industries, including the cosmetic industry for the preparation of cosmetic products, creams, gels, etc. The ingredient can be called "topical active ingredient" when its destination is a topical application. "Topical application" means an application that is intended to act where it is applied: skin, mucous, appendages. Topical application may be especially cosmetic, pharmaceutical or cosmeceutical.

In particular the combination of plant extracts according to the present invention can be used as a cosmetic active ingredient to improve the appearance and general condition of the skin and its annexes, in particular for the treatment and/or prevention of signs of aging and/or for the treatment and/or prevention of imperfections of skin and its appendages.

Therefore an object of the invention is the use of a cosmetic active ingredient comprising (i) an extract of *Astragalus membranaceus,* (ii) an extract of *Bupleurum falcatum,* and (iii) an extract of *Atractylodes macrocephalae* or of a composition comprising said ingredient in a physiological acceptable medium, for a cosmetic topical treatment for improving the appearance and general condition of the skin and its annexes, in particular for treating and/or preventing ageing signs and/or imperfections of the skin and its annexes.

"Skin" means according to the invention, the entire body surface, including the skin, mucous membranes and scalp.

"Appendages" means all skin annexes including nails, hair, eyelashes, eyebrows and body hair. "Improving the appearance and general condition of the skin and its annexes" means according to the invention acting both at preventive and curative levels on disorders and diseases, signs of deterioration, including fighting against aging and/or against the imperfections of the skin, mucous membranes or skin appendages.

"Treating and/or preventing the signs of aging" means preventing and/or limiting the arrival of cutaneous signs of aging such as fine lines, wrinkles and dry skin, loss of tone, elasticity, etc. It means, when the skin is already showing signs of cutaneous aging, correcting and/or reducing the signs of aging. Aging also affects the mucous membranes and skin appendages such as hair, body hair (including eyelashes and eyebrows) and nails, and the signs of aging can then be very different, but not less annoying.

"Treating and/or preventing or fighting against skin imperfections" means to prevent and/or limit the appearance of skin imperfections such as discontinuities in texture or tone, degradation of feelings of skin, mucous membranes or skin appendages, such as tightness, dryness, itching. It also includes, when the skin is already showing imperfections, treating and/or alleviating these imperfections.

"Disorders and diseases," signs of degradation of the appearance and general condition of the skin, mucous membranes or skin appendages, means according to the invention in particular:
❖ disorders associated with an increase of MMPs, collagen decrease, glycation increase, increase in free radicals, inflammation, degradation of the extracellular matrix, including:
   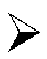 the emergence and development of texture discontinuities such as fine lines and wrinkles, the number and depth of wrinkles, particularly wrinkles arranged radially around mouth and/or eyes, especially crow's feet wrinkles, and/or located at the forehead, especially the so-called lion wrinkle, and/or placed horizontally on the forehead, located at the glabella, wrinkles and fine lines between the eyebrows, folds of the neck;
   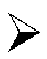 sagging of skin and subcutaneous tissues, sagging of neck skin, a withered and flaccid skin, also at the level of the forearm and thighs;
   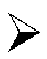 loss of firmness, tone and skin elasticity, ptosis, atony of skin texture, skin and cutaneous anisotropy;
   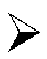 the thickness variation of epidermis and/or dermis, skin atrophy;
   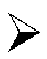 tightness loss, loss of skin resistance to deformation;
   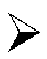 the appearance of under eye bags;
   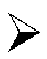 dry and rough appearance of the skin, roughness, reduction in the skin smoothness, change in skin texture;
   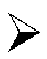 cutaneous dryness due in particular to sebaceous reduction and/or increase in insensible water loss of the skin, xerosis;
   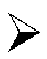 keratoses, abnormal differentiation, hyperkeratination, elastosis;
❖ disorders associated with increased pigmentation and inflammation, including:
   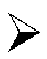 change of skin complexion, reducing the clarity and brightness of the skin tone, dullness, loss of uniformity of skin tone, pigmentation changes (hypo and hyper pigmentation), the appearance of colored or dark spots, local rednesses;
   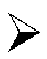 pigment spots, hyperkeratosis spots, UV-visible sunspots, lentigines, age spots;
   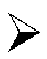 increased vascular signs (appearance of spider veins, angiomas);
❖ disorders associated with an increase in free radicals and inflammation, including:
   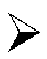 disorganisation of microcirculation of skin and fundamental tissues close to the skin;
   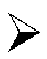 the appearance of irritated areas;
❖ disorders related to a combination of these factors, including:
   - desquamation increase;
   - pore size variation, enlarged pores;
   - pH change ;
   - hair color and texture change, hair loss, dull, altered hair;
   - unbalanced scalp;
   - brittle nails,
   - slowdown in the growth of hair and nails;
   - symptoms due to menopause;
   - and other histological changes in the *stratum corneum,* epidermis and dermis.

The studies detailed thereafter in the text show that the combination of plant extracts according to the invention strengthens the epidermis and improves the skin barrier formation over time, strengthens the epidermis /dermis connection, strengthens the dermal matrix, protects against pollution, and modulates pigment functions that make this combination a useful active ingredient component to improve the general condition of the skin and its annexes and to treat and/or prevent the signs of aging or blemishes of the skin and its annexes. The action of this combination may be preventively or curatively.

More particularly, the use of the combination of the extracts according to the present invention use is directed to a topical cosmetic:
- treatment for reducing the complexion inhomogeneity; and/or
- treatment for improving skin radiance; and/or
- anti-oxidant treatment; and/or
- treatment of skin dehydration; and/or
- treatment is for improving the mechanical properties of the skin, comprising firmness and elasticity; and/or
- treatment for improving the skin barrier function; and/or
- treatment for improving the dermo-epidermal junction.

Advantageously, the combination according to the invention will be present in a composition suitable for topical application to the skin or its appendages, containing a physiologically acceptable medium. These are in particular cosmetic or dermatological compositions.

The present invention also proposes a topical composition characterized in that it comprises the active ingredient according to the invention, in a physiologically acceptable medium.

The present invention also encompasses a topical composition comprising in a physiologically acceptable medium, at least the active ingredient according to the invention, and characterized in that the combination of plant extracts is present in sufficient amount to have an activity of enhancing skin barrier effect, of strengthening dermis and DEJ, a protective activity against the oxidation and pollution, and a modulating activity of pigment functions.

As explained above, the combination of plant extracts according to the invention has a remarkable action to improve the general condition of the skin, and in particular anti-aging.

Topical application may be cosmetic, dermocosmetic and/or dermopharmaceutical.

Preferably, the use is a cosmetic use. Cosmetic use means a use to improve the aesthetic appearance of the skin or its annexes, including delaying or diminishing the appearance of physiological changes that occur with age, of healthy persons. These changes can occur at 30 or 35 years, but are generally more pronounced after 40 years, and are increasing at 50 years and older.

According to other features of the invention, for the intented cosmetic topical use, the active ingredient or composition of the invention may include one or more additional active ingredients, to provide advantageously a product with a wider range of cosmetic properties. The active ingredients may for example be selected from the lightening, anti-redness, anti-spots, calming, for the treatment of sensitive or reactive skins, UV sunscreens, moisturizers, humectants, exfoliating, smoothing, toning, anti-aging (for reinforcing the activity), anti-wrinkles and fine lines, slimming, volumizing, improving mechanic and elastic properties, radiance, detoxifying actives, acting on cutaneous barrier, anti-acne, acting on sebum production, mating, unifying, anti-inflammatory, anti-oxidant, anti-radical, anti-glycation, propigmenting or depigmenting, depilatories, anti-regrowth, or promoting hair growth, eye contours (dark circle, under eye bags), promoting blood circulation, peptides, vitamins, etc.. These active ingredients can be obtained from plant materials, such as plant extracts or plant cell culture products plant or by fermentation.

More specifically, the combination of extracts according to the invention can be combined with at least one of the compounds selected from the compounds of B3 vitamin, compounds as niacinamide or tocopherol, retinoids compounds, such as retinol, hexamidine, α-lipoic acid, resveratrol or DHEA, peptides, including N-acetyl-Tyr-Arg-O-hexadecyl ester, Pal-VGVAPG (SEQ ID NO: 1), Pal-KTTKS (SEQ ID NO: 2), Pal-GHK, Pal-KMO₂K and Pal-GQPR (SEQ ID NO: 3), which are widely used active ingredients in topical cosmetic or dermo-pharmaceutical compositions.

The present invention will be better understood in light of the following description.

### DETAILED DESCRIPTION

### Composition preparation

"Physiologically acceptable medium" means according to the present invention, without limitation, an aqueous or hydroalcoholic solution, a water-in-oil emulsion, an oil-in-water emulsion, a micro-emulsion, an aqueous gel, an anhydrous gel, a serum, a dispersion of vesicles, or a powder.

"Physiologically acceptable" means that the compositions are suitable for topical or transdermal use, in contact with mucous membranes, appendages (nails, hair and body hair), scalp and skin of mammals, particularly human, compositions which may be ingested or injected into the skin, without risk of toxicity, incompatibility, instability, allergic response, and others.

This "physiologically acceptable medium" forms what is commonly called the excipient of the composition.

The effective amount of the active ingredient or the combination of extracts according to the invention, that is to say its dosage, depends on the destination of the topical composition, cosmetic, pharmaceutical or cosmeceutical.

The cosmetic, pharmaceutical or cosmeceutical effective amount according to the invention to be administered to treat a disorder or disease and its dosage depends on various factors, such as the age, the condition of the patient, the severity of the disorder or disease and the administration mode. An effective amount means a non-toxic amount enough to achieve the desired effect.

In a composition according to the invention, the active ingredient comprising the combination of extracts to be present in an effective amount, is generally present in an amount ranging from 0.0001% to 90% , preferably between 0.001% and 40%, more preferably between 0.01 and 10%, based on the total weight of the composition. The one skilled in the art is capable of adjusting the ingredient or extract content depending on the specific destination and the desired effect.

All percentages and ratios used herein are by weight of the total composition and all measurements are made at 25°C unless it is otherwise specified.

The choice of the excipient of the composition is made according to the constraints of the active ingredient or the combination of extracts (stability, solubility, etc.), and if necessary according to the intended galenic form envisaged afterwards for the composition.

The active ingredient comprising the combination of extracts according to the invention can be incorporated in the composition by means of an aqueous solution, or thanks to a conventional solubilizer, for example and without limiting to this list: ethanol, propanol, isopropanol, propylene glycol, glycerin, butylene glycol, or polyethylene glycol or any combination thereof. A powder support can also be used.

The compositions of the present invention are generally prepared by conventional methods well known to one skilled in the art for making topical or oral compositions and compositions for injection. Such methods may involve a mixture of ingredients in one or more steps to obtain a uniform state, with or without heating, cooling, etc.

All galenic forms that can contain the active ingredient according to the invenion can be used, i.e., creams, lotions, milk or cream ointments, gels, emulsions, dispersions, solutions, suspensions, cleansers, foundations, anhydrous preparation (skicks for example lip balm, body and bath oils), shower and bath gels, shampoos and hair care lotions, milks or creams for skin and hair cares, cleansing lotions or milks, sunscreen lotions, milks or creams, artificial tanning lotions, milks or creams, pre-shave, shaving or after-shave creams, foams, gels or lotions, make-up, lipsticks, mascaras or nail varnishes, skin "essences," serums, adhesive or absorbent materials, transdermal patches, emollient powders, lotions, milks or creams, sprays, oils for the body and the bath, foundation tint bases, pomade, emulsion, colloid, compact or solid suspension, pencil, sprayable or brossable formulation, blush, red, eyeliner, lip liner, lip gloss, facial or body powder, styling foams or gels, nail conditioner, lip balms, skin conditioners, moisturizers, hair sprays, soaps, body exfoliants, astringents, depilatories and permanent waving solutions, antidandruff formulations, anti-sweat and antiperspirant compositions, nose sprays etc. These compositions can also be presented in the form of lipsticks intended to apply color or to protect the lips from cracking, or of make-up products for the eyes or tints and tint bases for the face. Compositions in accordance with the invention include cosmetics, personal care products and pharmaceutical preparations. A composition in the form of foam or in the form of compositions for aerosol also including a propellant agent under pressure can be envisaged.

Cosmetic compositions according to the invention may also be for orodental use, for example, toothpaste. In that case, the compositions may contain the usual adjuvants and additives for compositions for oral use and, in particular, surfactants, thickening agents, moisturizing agents, polishing agents such as silica, various active substances such as fluorides, particularly sodium fluoride, and, possibly, sweetening agents such as saccharin sodium.

The active ingredient or composition within the scope of the present invention may be used in the form of solution, dispersion, emulsion, paste, or powder, individually or as a premix or in vehicles individually or as a premix in vectors such as macro-, micro-, or nanocapsules, macro-, micro- or , nanospheres, liposomes, oleosomes or chylomicrons, macro-, micro-, or nanoparticles or macro-, micro or nanosponges, , micro or nano emulsions or adsorbed on organic polymer powders, talcs, bentonites, spores or exines, and other inorganic or organic supports.

The active ingredient or the composition according to the present invention may be used in any form whatsoever, in a form bound to or incorporated in or absorbed in or adsorbed on macro-, micro-, and nanoparticles, or macro-, micro-, and nanocapsules, for the treatment of textiles, natural or synthetic fibers, wools, and any materials that may be used for clothing or underwear for day or night intended to come into contact with the skin, handkerchiefs or cloths, to exert their cosmetic effect via this skin/textile contact and to permit continuous topical delivery.

The invention also discloses a woven or nonwoven material comprising an ingredient or a composition according to the invention for use or a method according to the invention.

The pH of a composition according to the invention, when it comprises at least an aqueous phase (e.g. aqueous solutions, emulsions, ...), is preferably between 4 and 9, preferably between 4 and 7, advantageously from 5 to 6, and particularly a pH of 5.5.

### Cosmetic treatment method

The present invention also concerns a topical treatment method to improve the appearance and general condition of the skin and its annexes, comprising the topical application to the skin of a subject in need thereof of an effective amount of the invention active ingredient, combination, or of a composition as recited above.

The invention therefore relates to a cosmetic topical application of the active ingredient, combination or composition of the invention to improve the appearance and general condition of the skin and its annexes and especially for treating and/or preventing the signs of aging and/or skin imperfections of skin, mucous membranes or appendages.

"Topical treatment" or "topical use" means an application that is intended to act where it is applied: skin, mucosa, skin appendages. A topical application can particularly be cosmetic, pharmaceutical or cosmeceutical.

The topical use of the invention can specifically target the chronological aging, hormonal aging, actinic aging (photodamage) or aging due to environmental effects.

One of the preferred modes of topical cosmetic use of the combination according to the invention, or of a composition according to the invention, is intended, as a preventive or curative improvement of skin tone, of the radiance, of the firmness of the skin, for reducing wrinkles and fine lines, skin dryness, the imbalance of scalp, hair loss and brittle nails.

Owing to its properties, the combination of the invention is also particularly suitable for the care and/or treatment of sensitive skin, sensitized skin, and/or reactive skin. The active ingredient and composition according to the invention are also suitable for a use and a cosmetic method on Asian skin.

The invention also concerns a cosmetic method for improving the general state of the skin and its annexes comprising topically applying on the skin of a subject in need thereof an effective amount of an ingredient or a composition according to the invention characterized in that the application brings at least one of the following effects:
a. Treats and/or prevent the cutaneous ageing signs,
b. Treats and/or prevent the defects,
c. Stimulates keratinocyte differentiation,
d. Stimulates laminin synthesis by keratinocytes,
e. Stimulates hyaluronic acid synthesis by keratinocytes,
f. Stimulates collagen synthesis,
g. Protects against lipid peroxydation,
h. Decreases melanin synthesis,
i. Strengthens the cutaneous barrier effect,
j. Strengthens dermis,
k. Strengthens DEJ,
l. Protects against oxidation,
m. Potects against pollutions,
n. Modulate pigmentary fonctions.

More particularly, the method of the invention is:
- for reducing the complexion inhomogeneity; and/or
- for improving skin radiance; and/or
- an anti-oxidant treatment; and/or
- a treatment of skin dehydration; and/or
- for improving the mechanical properties of the skin, comprising firmness and elasticity; and/or
- for improving the skin barrier function; and/or
- for improving the dermo-epidermal junction.

Improvements in the appearance and general condition of the skin, of mucous membranes, and of appendages can be obtained by topical application of these compositions on a regular basis such as daily or twice daily.

In the methods according to the invention, the synergistic combination or the composition containing it, will be applied preferably topically on the skin, mucous membranes and/or keratinous fibers. In the case of a cosmetic treatment to delay or prevent the signs of aging, the application may be for example renewed before, during and/or after exposure to UV radiations.

Results can be observed from the application of the cosmetic treatment. However, the cosmetic treatment according to the invention may be extended for a 2, 4 or 8 weeks, or even months.

The combination or composition containing it is preferably applied on skin areas affected by the disorder against which fighting is desired. These areas include face skin, forehead, lips, neck, chest, arms and hands, but also body areas like legs, thighs, knees, feet, chest, back, buttocks, or abdomen or any area to be treated.

The practitioner will appreciate the topical cosmetic treatment that will include a composition containing the combination of extracts; this treatment can be achieved for example by applying topically to the composition described in the present invention, according to a method usually used to apply such a composition. The topical composition is preferably applied to the face and neck, but can be applied to any part of skin requiring an aesthetic improvement, where the composition remains on the skin area to be treated, and preferably is not removed or flushed from the skin. For example, for a face cosmetic treatment, the European Cosmetics Directive has set a standard amount for applying a cream of 2.72 mg/cm²/day/person.

It is also to be understood that, as used herein, the terms treating and treatment include and encompass the reduction, improvement, progress, relief, and/or elimination of dermatological effects, including aging.

One of the major advantages of the present invention resides in the ability whenever necessary or desirable to be able to apply local selective "gentle" treatments through this topical, non-invasive method of application. In the case of anti-wrinkle use for example it may be applied very locally using a syringe or micro-canula.

It is also possible, however, to consider a composition containing the ingredient according to the invention intended to be injected subcutaneously.

According to other specific features, the cosmetic treatment method according to the invention can be combined with one or more other treatment methods targeting the skin such as lumino-therapy, heat or aromatherapy treatments.

According to the invention, devices with several compartments or kits may be proposed to apply the method described above which may include for example and non-restrictively, a first compartment containing a composition comprising the combination of extracts or one of the extracts of the invention, and in a second compartment a composition containing another active ingredient and/or excipient or one of the extracts of the inventon, the compositions contained in the said first and second compartments in this case being considered to be a combination composition for simultaneous, separate or stepwise use in time, particularly in one of the treatment methods recited above.

For a cosmeceutical or pharmaceutical application, the composition according to the invention is an adapted form that can be ingested.

The invention also proposes a composition comprising the combination of extracts according to the invention for a therapeutical treatment of degraded skin, mucous membranes and skin appendages.

In cosmetics in particular, applications can be offered for example in product lines for moisturizing, make up removal, cleansing, anti-aging, antioxidant, protective, restorative (hands, feet, lips), contours (face, eyes, neck, lips), the makeup-care of the skin and the appendages, including eyelashes, lip products, sunscreens, sculpting, plumping, volumizing (e.g. on the hands, chest, breasts), hair products, nail care etc..

### Additional ingredients

The CTFA International cosmetic ingredient dictionary & handbook (13th Ed. 2010) (published by the Cosmetic, Toiletry, and Fragrance Combination, Inc., Washington, D.C.) describes a non-limited wide variety of cosmetic and pharmaceutical ingredients conventionally used in the skin care industry that can be used as additional ingredients/compounds in the compositions for the present invention. Examples of these ingredient classes include, but are not limited to: healing agents, skin anti-aging agents, anti-wrinkle agents, anti-atrophy agents, skin moisturizing agents, skin smoothing agents, antibacterial agents, anti-parasitic agents, antifungal agents, fungicidal agents, fungistatic agents, bactericidal agents, bacteriostatic agents, antimicrobial agents, anti-inflammatory agents, anti-pruriginous agents, anesthetic agents, antiviral agents, keratolytic agents, free radicals scavengers, anti-seborrhea agents, antidandruff agents, the agents modulating differentiation, proliferation or pigmentation of the skin and agents accelerating penetration, desquamating agents, melanin synthesis stimulating or inhibiting agents, whitening, depigmenting or lightening agents, pro-pigmenting agents, self-tanning agents, NO-synthase inhibiting agents, antioxidants, free radical scavengers and/or agents against atmospheric pollution, reactive carbonyl species scavengers, anti-glycation agents, tightening agents, agents stimulating the synthesis of dermal or epidermal macromolecules and/or capable of inhibiting or preventing their degradation, such as for example collagen synthesis stimulating agents, elastin synthesis stimulating agents, decorin synthesis stimulating agents, laminin synthesis stimulating agents, defensin synthesis stimulating agents, chaperone synthesis stimulating agents, aquaporin synthesis stimulation agents, hyaluronic acid synthesis stimulating agents, fibronectin synthesis stimulating agents, sirtuin synthesis-stimulating agents, agents stimulating the synthesis of lipids and components of the stratum corneum (ceramides, fatty acids, etc.), collagen degradation inhibiting agents elastin degradation inhibiting agents, agents stimulating fibroblast proliferation, agents stimulating keratinocyte proliferation, adipocyte proliferation stimulating agents, melanocyte proliferation stimulating agents, keratinocyte differentiation stimulating agents, adipocyte differentiation stimulating agents, acetylcholinesterase inhibiting agents, glycosaminoglycan synthesis stimulating agents, DNA repair agents, DNA protecting agents, anti-itching agents, agents for the treatment and/or care of sensitive skin, firming agents, anti-stretch mark agents, astringent agents, sebum production regulating agents, dermo-relaxing agents, healing adjuvant agents, re-epithelialization stimulating agents, re-epithelialization co-adjuvant agents, cytokine growth factors, calming agents, anti-inflammatory agents, agents acting on capillary circulation and/or microcirculation, angiogenesis stimulating agents, vascular permeability inhibiting agents, agents acting on cell metabolism, agents able to improve dermal-epidermal junction, agents inducing hair growth, hair growth inhibiting or retardant agents, muscle relaxants agents, antipollution and/or anti-free radical agents, lipolysis stimulating agents, slimming agents, anti-cellulite agents, agents acting on the microcirculation, agents acting on the energy metabolism of the cells, cleaning agents, hair conditioning agents, hair styling agents, hair growth promoters, sunscreen agents, total sunscreen agents, make-up agents, detergents, pharmaceutical products, emulsifiers, emollients, organic solvents, antiseptic agents, deodorant actives, physiologically acceptable carriers, surfactants, abrasives, absorbents, aesthetic components such as fragrances, pigments, dyes, colorants, natural colorants, essential oils, touch agents, cosmetic astringents, anti-acne agents, anti-coagulation agents, antifoaming agents, antioxidants, binders, biological additives, enzymes, enzymatic inhibitors, enzyme-inducing agents, coenzymes, chelating agents, plant extracts, plant derivatives, essential oils, marine extracts, agents obtained from a bio-fermentation or a biotechnological process, mineral salts, cell extracts, sunscreens (organic or mineral photoprotective agents active against ultraviolet A and/or B rays), ceramides, peptides, buffers, volumizing agents, chelating agents, chemical additives, colorants, cosmetic biocides, denaturants, medical astringents, external analgesics, film formers, such as polymers, for exacerbing film-forming properties and substantivity of the composition, quaternary derivatives, substantivity increasing agents, opacifying agents, pH adjusters and regulators (e.g. triethanolamine), propellants, reducing agents, sequestrants, decoloring and/or lightening agents, skin-conditioning agents (e.g., humectants, including miscellaneous and occlusive), moisture retaining agents, alphahydroxyacids, betahydroxyacids, moisturizers, epidermal hydrolytic enzymes, healing and/or calming agents, skin treating agents, anti-wrinkle agents, agents that reduce or treat bags under the eyes, exfoliating agents, thickeners, softening agents, gelling polymers, vitamins and their derivatives, wetting agents, peeling agents, soothing agents, curative agents of the skin, lignans, preservatives (i.e. phenoxyethanol and parabens), anti UV, cytotoxic agents, anti-neoplastics, viscosity modifiers, non-volatile solvents, pearling agents, anti-perspirant agents, depilatories, vaccine, perfumed water, skin restructuring agent (i.e. Siegesbeckia orientalis extract), excipients, charges, minerals, anti-mycobacterial agents, anti-allergenic agents, HI or H2 antihistaminics, anti-irritants, agents stimulating the immune system, agents inhibiting the immune system, insect repellents, lubricants, pigments or dyes, hypopigmentation agents, preservatives, light stabilizers, and mixtures thereof, as long as they are physically and chemically compatible with the other ingredients of the composition and especially with the active ingredients of the present invention. Also the nature of these additional ingredients should not unacceptably alter the benefits of the active ingredients of the invention. These additional ingredients can be synthetic or natural such as plants extracts, or come from a bio-fermentation process. Additional examples can be found in the CTFA Cosmetic Ingredient Handbook.

Such additional active ingredient/compound can be selected from the group consisting of: sugar amines, glucosamine, D-glucosamine, N-acetyl glucosamine, N-acetyl-D-glucosamine, mannosamine, N-acetyl mannosamine, galactosamine, N-acetyl galactosamine, B3 vitamin and its derivatives, niacinamide, sodium dehydroacetate, dehydroacetic acid and its salts, phytosterols, salicylic acid compounds, hexamidines, dialkanoyl hydroxyproline compounds, soy extracts and derivatives, equol, isoflavones, flavonoids, phytantriol, farnesol, geraniol, bisabolol, peptides and their derivatives, di-, tri-, tetra-, penta-, and hexapeptides and their derivatives, KTTKS (SEQ ID NO: 4), Pal-KTTKS (SEQ ID NO: 2), carnosine, N-acyl amino acid compounds, retinoids, retinyl propionate, retinol, retinyl palmitate, retinyl acetate, retinal, retinoic acid, water-soluble vitamins, ascorbates, C vitamin, ascorbyl glucoside, ascorbyl palmitate, magnesium ascorbyl phosphate, sodium ascorbyl phosphate, vitamin B and their salts and derivatives, B1 vitamin, B2 vitamin, B6 vitamin, B12 vitamin, provitamins and their salts and derivatives, K vitamin and derivatives, pantothenic acid and its derivatives, pantothenyl ethyl ether, panthenol and derivatives, dexpanthenol, biotin, amino acids and their salts and derivatives, water soluble amino acids, asparagine, alanine, indole, glutamic acid, water insoluble vitamins, A vitamin, E vitamin, F vitamin, D vitamin and mono-,di-, and tri-terpenoids compounds, beta-ionol, cedrol, and their derivatives, water insoluble amino acids, tyrosine, tryptamine, particulate materials, butylated hydroxytoluene, butylated hydroxyanisole, allantoin, tocopherol nicotinate, tocopherol, tocopherol esters, palmitoyl-Gly-His-Lys (Pal-GHK), phytosterol, hydroxy acids, glycolic acid, lactic acid, lactobionic acid, keto acids, pyruvic acid, phytic acid, lysophosphatidic acid, stilbenes, cinnamates, resveratrol, kinetin, zeatin, dimethylaminoethanol, natural peptides, soy peptides, salts of sugar acids, Mn gluconate, Zn gluconate, piroctone olamine, 3,4,4'-trichlorocarbanilide, triclocarban, Zn pyrithione, hydroquinone, kojic acid, ascorbic acid, magnesium ascorbyl phosphate, ascorbyl glucoside, pyridoxine, aloe vera, terpene alcohols, allantoin, bisabolol, dipotassium glycyrrhizinate, glycerol acid, sorbitol acid, pentaerythritol acid, pyrrolidone acid and salts, dihydroxyacetone, erythrulose, glyceraldehyde, tartaraldehyde, clove oil, menthol, camphor, eucalyptus oil, eugenol, menthyl lactate, witch hazel distillate, eicosene and vinyl pyrrolidone copolymer, iodopropyl butylcarbamate, a polysaccharide, an essential fatty acid, a salicylate, glycyrrhetinic acid, carotenoids, ceramides and pseudo-ceramides, a lipid complex, oils in general of natural origin such shea butter, apricot oil, onagre oil, prune oil, palm oil, monoi oil, hydroquinone, HEPES, procysteine, O-octanoyl-6-D-maltose, the disodium salt of methylglycinediacetic acid, steroids such as diosgenin and derivatives of DHEA, DHEA or dehydroepiandrosterone and/or a precursor or chemical or biological derivative thereof, N-ethyloxycarbonyl-4-para-aminophenol, blueberries extracts, phytohormones, extracts of the yeast Saccharomyces cerevisiae, extracts of algae, extracts of soyabean, lupin, maize and/or peas, alverine and its salts, in particular alverine citrate, extract of butcher's broom and of horse chestnut, octadecenedioic acid, analogs and derivatives, and mixtures thereof, a metalloproteinase inhibitor.

Further skin care and hair care active ingredients that are particularly useful combined with the composition can be found in SEDERMA commercial literature and on the website www.sederma.fr. The following commercial actives can also be mentioned, as examples: betain, glycerol, Actimoist Bio 2™ (Active organics), AquaCacteen™ (Mibelle AG Cosmetics), Aquaphyline™ (Silab), AquaregulK™ (Solabia), Carciline™ (Greentech), Codiavelane™ (Biotech Marine), Dermaflux™ (Arch Chemicals, Inc), Hydra'Flow™ (Sochibo), Hydromoist L™ (Symrise), RenovHyal™ (Soliance), Seamoss™ (Biotech Marine), Essenskin™ (Sederma), Moist 24™ (Sederma), Argireline™ (trade name of the acetyl hexapeptide-3 of Lipotec), spilanthol or an extract of *Acmella oleracea* known under the name Gatuline Expression™, an extract of *Boswellia serrata* known under the name Boswellin™, Deepaline PVB™ (Seppic), Syn-AKE™ (Pentapharm), Ameliox™, Bioxilift™ (Silab), Juvinity™ (Sederma), Revidrate™ (Sederma), Resistem™ (Sederma), Legance™ (Sederma), Beautifeye™ (Sederma), Prodizia™ (Sederma), or mixtures thereof.

Among other plant extracts which can be combined with the composition of the invention, there may more particularly be mentioned extracts of Ivy, in particular English Ivy (*Hedera Helix*), of *Bupleurum chinensis,* of *Bupleurum Falcatum,* of arnica (*Arnica Montana L*), of rosemary (*Rosmarinus officinalis* N), of marigold (*Calendula officinalis*), of sage (*Salvia officinalis L*), of ginseng (*Panax ginseng*), of ginko biloba, of St.-John's-Wort (*Hyperycum Perforatum*), of butcher's-broom (*Ruscus aculeatus L*), of European meadowsweet (*Filipendula ulmaria L*), of big- flowered Jarva tea (*Orthosiphon Stamincus Benth*), of algae (*Fucus Vesiculosus*), of birch (*Betula alba*), of green tea, of cola nuts (*Cola Nipida*), of horse-chestnut, of bamboo, of *Centella asiatica,* of heather, of fucus, of willow, of mouse-ear, of escine, of cangzhu, of *chrysanthellum indicum,* of the plants of the *Armeniacea* genus, *Atractylodis Platicodon, Sinnomenum, Pharbitidis, Flemingia,* of *Coleus* such as *C*. *Forskohlii, C. blumei, C. esquirolii, C. scutellaroides, C. xanthantus* and *C*. *Barbatus,* such as the extract of root of *Coleus barbatus,* extracts of *Ballote,* of *Guioa,* of *Davallia,* of *Terminalia,* of *Barringtonia,* of *Trema,* of *antirobia, cecropia, argania, dioscoreae* such as *Dioscorea opposita* or Mexican, extracts of *Ammi visnaga,* of *Siegesbeckia,* in particular *Siegesbeckia orientalis,* vegetable extracts of the family of *Ericaceae,* in particular bilberry extracts (*Vaccinium angustifollium*) or *Arctostaphylos uva ursi, aloe vera,* plant containing sterols (e.g., phytosterol), Manjistha (extracted from plants of the genus *Rubia,* particularly *Rubia Cordifolia*), and Guggal (extracted from plants of the genus *Commiphora,* particularly *Commiphora Mukul*), kola extract, chamomile, red clover extract, Piper methysticum extract (Kava Kava™ from SEDERMA), *Bacopa monieri* extract (Bacocalmine™ from SEDERMA) and sea whip extract, extracts of *Glycyrrhiza glabra,* of mulberry, of *melaleuca* (tea tree), of *Larrea divaricata,* of *Rabdosia rubescens,* of *Euglena gracilis,* of *Fibraurea recisa Hirudinea,* of *Chaparral Sorghum,* of sun flower extract, of *Enantia chlorantha,* of Mitracarpe of *Spermacocea* genus, of *Buchu barosma,* of *Lawsonia inermis L.,* of *Adiantium Capillus-Veneris L.,* of *Chelidonium majus,* of *Luffa cylindrica,* of Japanese Mandarin (*Citrus reticulata Blanco* var. *unshiu*), of *Camelia sinensis,* of *Imperata cylindrica,* of *Glaucium Flavum,* of *Cupressus Sempervirens,* of *Polygonatum multiflorum,* of *loveyly hemsleya,* of *Sambucus Nigra,* of *Phaseolus lunatus,* of *Centaurium,* of *Macrocystis Pyrifera,* of *Turnera Diffusa,* of *Anemarrhena asphodeloides,* of *Portulaca pilosa,* of *Humulus lupulus,* of *Coffea Arabica,* of *Ilex Paraguariensis,* of *albizzia julibrissin, Oxydendron arboretum* or of *Zingimber Zerumbet Smith.*

The compositions of the present invention may include peptides, including, without limitation, the di-, tri-, tetra-, penta-and hexapeptides and their derivatives. According to a particular embodiment, the concentration of the additional peptide, in the composition, ranges from 1x10⁻⁷% and 20%, preferably from 1x10⁻⁶% and 10%, preferably between 1x10⁻⁵% and 5%, by weight.

According to the present invention, the term "peptide" refers to peptides containing 10 amino acids or less, their derivatives, isomers and complexes with other species such as a metal ion (e.g. copper, zinc, manganese, magnesium, and others). The term "peptides" refers to both natural peptides and synthetic peptides. It also refers to compositions that contain peptides which are found in nature, and/or are commercially available.

Suitable dipeptides for use within the scope of the present invention include but are not limited to carnosine (beta-AH), YR, VW, NF, DF, KT, KC, CK, KP, KK or TT. Non limitative suitable tripeptides for use herein include, but are not limited to RKR, HGG, GHK, GKH, GGH, GHG, KFK, KPK, KMOK, KMO₂K or KAvaK. Non limitative suitable tetrapeptides for use herein include but are not limited to RSRK (SEQ ID NO: 5), GQPR (SEQ ID NO: 6) or KTFK (SEQ ID NO: 7). Non limitative suitable pentapeptides include, but are not limited to KTTKS (SEQ ID NO: 4) and hexapeptides include but are not limited to GKTTKS (SEQ ID NO: 8) and VGVAPG (SEQ ID NO: 9).

Other suitable peptides for use in the context of the present invention include, but are not limited to: lipophilic derivatives of peptides, preferably palmitoyl derivatives, and metal complexes as aforementioned (e.g. copper complex of the tripeptide HGG). Preferred dipeptide derivatives include N-Palmitoyl-beta-Ala-His, N-Acetyl-Tyr-Arg-hexadecylester (Calmosensine™, Idealift™ from Sederma). Preferred tripeptide derivatives include in particular the N-Palmitoyl-Gly-Lys-His, and Pal-Gly-His-Ly, (Pal-GKH and Pal-GHK from Sederma), the copper derivative of HGG (Lamin™ from Sigma), Lipospondin (N-Elaidoyl-KFK) and its analogs of conservative substitution, N-Acetyl-RKR-NH₂ (Peptide CK+), N-Biot-GHK (from Sederma), Pal-KMO₂K (Sederma) and derivatives thereof. Suitable tetrapeptide derivatives for use according to the present invention include, but are not limited to, N-palmitoyl-GQPR (SEQ ID NO: 3) (from Sederma), suitable pentapeptide derivatives for use herein include, but are not limited to, N-Palmitoyl-KTTKS (SEQ ID NO: 2) (available as Matrixyl™ from Sederma), N-Palmitoyl-Tyr-Gly-Gly-Phe-X (SEQ ID NO: 10) with X Met or Leu or mixtures thereof. Suitable hexapeptide derivatives for use herein include, but are not limited to, N-Palmitoyl-VGVAPG (SEQ ID NO: 1) and derivatives thereof. The mixture of Pal-GHK and Pal-GQPR (SEQ ID NO: 3) (Matrixyl™ 3000, Sederma) can also be mentioned.

The preferred compositions commercially available containing a tripeptide or a derivative include Biopeptide-CL™, Maxilip™, Biobustyl™, Procapil™ and Matrixyl™synthe'6™ of Sederma. The compositions commercially available preferred sources of tetrapeptides include Rigin™, Eyeliss™, Matrixyl™ Reloaded and Matrixyl 3000™ which contain between 50 and 500 ppm of Palmitoyl-GQPR (SEQ ID NO: 3) and carrier, proposed by Sederma.

The following marketed peptides can be mentioned as well as additional active ingredients: Vialox™, Syn-ake™ or Syn-Coll™ (Pentapharm), Hydroxyprolisilane CN™ (Exsymol), Argireline™, Leuphasyl™, Aldenine™, Trylgen™, Eyeseryl™, Serilesine™ or Decorinyl™ (Lipotec), Collaxyl™ or Quintescine™ (Vincience), BONT-L-Peptide™ (Infinitec Activos), Cytokinol™LS (Laboratoires Serobiologiques/Cognis), Kollaren™, IP2000™ or Meliprene™ (lnstitut Européen de Biologie Cellulaire), Neutrazen™ (Innovations), ECM-Protect™ (Atrium Innovations), Timp-Peptide™ or ECM Moduline™ (Infinitec Activos).

The following examples describe and illustrate some aspects of the invention. They should not be seen as limiting the disclosure, as they mainly provide useful information for understanding and implementation.

### A) Preparation example of the combination of extracts, active ingredient of the invention

Plant roots are extracted with an alcohol or mixture of alcohols, preferably ethanol. After removal of the alcohol and filtration, the residue is taken up with a glycerin/water mixture 50/50 w/w. After a homogenisation step at 40°C followed by a final filtration step, the combination of extracts forming the active ingredient according to the invention is ready for use.

### In vitro studies

The active ingredient of the invention has a number of remarkable effects presented below. The extract concentrate prepared in above Example A) and diluted in aqueous vehicles, cell culture media type was *in vitro* tested and showed a various activities related to several areas of skin physiology.

### 1. Reinforcement of the cutaneous barrier effect

The combination of the extracts of the three plants of the invention improves the barrier effect related to the *stratum corneum,* making it more hydrated, more flexible and giving it a better resistance to external stress. This effects has been shown in three tests.

### 1.1. Keratinocytes differentiation study

Confluent human keratinocytes of Chinese origin were given increasing concentrations of the combination of extracts in an appropriate culture medium in order to study their differentiation. The differentiation was visually assessed at 3 and 7 days. Fluorescent labeling of neutral lipids and sphingosine-[trans-D-erythro-2-Amino-4-octadecene-1,3-diol], ceramide precursor was performed and quantified after 7 days of contact.

Visually, an acceleration of the differentiation caused by the combination of extracts in a contact-time and concentration-dependent manner is observed. As early as three days after keratinocytes came into contact, a stimulation of the formation of structures typical of the upper layers of a epidermis is noted (presence of branched structures characteristics of the proteo-lipidic and multilayer rigid matrix of the horny envelope), at 10 and mainly 30ppm of the combination of extracts. This effect is even more pronounced at 7 days.

These structures are visible in the presence of the combination of extracts of the invention, but were not observed in keratinocyte layers in contact with the extracts tested separately, at the same time. This therefore reflects a synergy between the three plants, giving a great cosmetic interest to this combination.

The labeling of neutral lipids (class to which ceramides belong, essential components of the *stratum corneum,* the culmination point of differentiation) on the same cellular layers confirms the visual observation and show an acceleration of differentiation in the presence of the combination of extracts (20% of labelling at 10ppm and +80% at 30ppm).

### 1.2. Effect on the YY1 protein

The keratinocytic over expression induced by YY1 moderates their proliferation in any skin type and thins the *stratum corneum.* It also inhibits the calcium-triggered terminal differentiation of keratinocytes by preventing the transcription of loricrin, a key component of the cornified envelope. YY1 is highly expressed in the keratinocytes of the basal layer, thereby ensuring that equilibrium is maintained in this layer in a poorly differentiated state. YY1 expression diminishes in the higher layers, which causes differentiation and barrier formation.

Confluent human keratinocytes were put into contact with combination of extracts in an appropriate culture medium. This test required large quantities of cells given the weak expression of the YY1 to be extracted from the cell layers and assessed by ELISA.

A decreased expression of YY1 is indeed observed at the differenciation stage on the control. The trial performed with the combination of extracts, in contact with keratinocytes for four days demonstrates that the combination of extracts significantly reduced YY1 (-34% at 30ppm, *p<0.01 vs* control). These two results (visual and biochemical) demonstrate that the combination of extracts stimulates the keratinocyte differentiation.

### 1.3. Effect on laminins and hyaluronic acid

These two components are synthesized by keratinocytes and fibroblasts and are essential for skin balance. Laminins together with a collagen IV form a network which constitutes the skeleton of the basal membrane. This membrane is the physical support of epidermis, these cells adhering thereto. It provides skin firmness and good communication between dermis and epidermis. Hyaluronic acid has an already well known role on skin hydration and elasticity.

Non-confluent human keratinocytes received escalating concentrations of the combination of extracts in an appropriate culture medium. At the end of this contact period, two assessments of laminin and hyaluronic acid were realized through ELISA. In parallel, the number of cells was estimated through the method using Hoescht 33258.

The results indicate that the combination of extracts stimulates the production of these 2 essential skin components: laminins (+42% at 10ppm and +125% at 20ppm; *p<0.01 vs*. control) as well as hyaluronic acid (+24% at 10ppm and +117% at 20ppm; *p<0.01 vs*. control).

Furthermore, in this type of study, stimulation of the proliferation of non-confluent keratinocytes is observed at all concentrations of the combination of extracts (+78% at 10ppm and +33% at 20ppm; *p<0.01 vs*. control). Thus, thanks to the combination of extracts a large number of keratinocytes can differentiate. The skin barrier is also enhanced by this way.

This stimulation effect on proliferation is not observed in non-confluent keratinocytes in contact with the extracts tested separately, in the same culture conditions. This reflects also here a synergy between the three plants.

### 2. Dermis and dermal-epidermal junction (DEJ) reinforcement

### 2.1. Effect on collagène IV

Collagen IV, as laminins, is an essential component of the basement membrane, which separates epidermis from dermis, allowing both cohesion of the assembly while maintaining a good communication. It also allows anchorage of the epidermis cells, which provides firmness.

After being anchored, normal human dermal fibroblasts (NHDF) received escalating concentrations of combination of extracts in an appropriate culture medium. After this contact, the culture supernatants were sampled and collagen IV levels were determined through ELISA. The results were compared to the control (without the combination of extracts). As for the keratinocytes, an estimation of the number of cells was performed using the Hoechst method 33258.

These results indicate that the combination of extracts stimulates the collagen IV synthesis (+28% at 10ppm and +51% at 15ppm; *p<0.01 vs*. control). It thus helps to reinforce the links between the two skin layers.

### 2.2. Effect on collagen I

The extracellular matrix is the cornerstone of maintaining skin dynamics and dermis viscoelastic properties. The components of this matrix undergo qualitative and quantitative structural modifications over time and under the effect of free radical-producing processes (mainly induced by the sun, pollution and unhealthy lifestyles), leading to skin aging and the appearance of wrinkles.

Collagen I is a crucial element for the balance of this matrix. Its production declines with age and its fibers are broken down by endogenous and exogenous radical processes. UVs also stimulate the synthesis of certain proteolytic enzymes that degrade the extracellular matrix proteins. This results in a decrease in the skin firmness and elasticity. It is therefore important to stimulate the synthesis of collagen I.

After being anchored, normal human dermal fibroblasts (NHDF) received escalating concentrations of the combination of extracts in an appropriate culture medium. After this contact, the culture supernatants were sampled and collagen I levels were determined through ELISA. The results were compared to the controls. The number of cells was estimated by the Hoechst 33258 method, and the observed values were reduces to the same cell quantity. These results were completed with another study that used direct immunofluorescent staining (IMF) of the collagen I deposited by NHDFs to form their extracellular matrix.

**Table 1:**

| **Collagen I (ELISA); n=5** | **% Change** |
|---|---|
| Negative control | Reference |
| 10ppm of the combination of extracts | +60%; *p<0.01* |
| 15ppm of the combination of extracts | +92%; *p<0.01* |

**Table 2:**

| **Collagen I (IMF); n=3** | **% Change** |
|---|---|
| Negative control | Reference |
| 10ppm of the combination of extracts | +190%; *p<0.01* |
| 15ppm of the combination of extracts | +237%; *p<0.01* |

These two complementary trials demonstrate that the combination of extracts can increase the synthesis of collagen I by normal dermal human fibroblasts in a dose-dependent way. Surprisingly, a synergy between the three plants for this activity has been demonstrated. Indeed, none of the extracts tested separately and under the same culture conditions on fibroblasts produces stimulation of the released collagen.

### 3. Protection against oxidation and pollutions

The skin is the organ most exposed to environmental conditions like the sun and various pollutants. Cosmetics is constantly looking for products that can prevent adverse effects on the skin of these agents.

### 3.1. Effect on lipid peroxydation

Artificial membranes acting as cell models were formed using phospholipids. These membrane models (n=3/conditions) were exposed to artificial sun treatment in the form of pro-oxidising UVA. The combination of extracts was put into contact with these membranes either before, during and after UVA radiation (protective effect) or only after radiation (restorative effect). The formation of precursors that signal the final alteration of lipids was followed by quantification of the formation of conjugated dienes. The results were compared with those of cases not receiving the combination of extracts.

For the protective effect, the inhibition of the diene formation is of -48% at 10ppm of the combination of extracts and of -64% at 30ppm (*p<0.01 vs*. control).

For the restorative effect, the inhibition of the diene formation is of -43% at 10ppm of the combination of extracts and of -71% at 30ppm (*p<0.01 vs*. control).

### 3.2. Effect on the oxygenated free radical production

In the following two trials, the oxidative stress was modeled on human fibroblasts: either with hydrogen peroxide (H₂O₂) (1^{st} trial), or with pollutants extracted from cigarette smoke (2^{nd} trial). In both models, the stress agent will generate free radicals in the cells. Intracellular probe that fluoresces in the presence of these free radicals will quantify them. The obtained result is reduced to the number of cells evaluated by the Hoechst method.

In both cases 30ppm of the combination of extracts can reduce the effects of the oxidative stress: -72% in the case of H₂O₂; -36% in the case of cigarette smoke. In both cases with *p<0.01 vs*. control. Without stress, the combination of extracts reduces also the oxygenated free radical basal concentration of about 30% (*p<0.01 vs*. control).

### 4. Modulation of the pigment functions

Some skin mark prematurely of pigment irregularities leading to heterogeneity and color defects. Normal human melanocytes were contacted with the combination of extracts for 10 days in a medium allowing their survival. After the contact, the melanin was extracted from the cells and assayed by spectrophotometry. The viability estimation of the cells was estimated in parallel. With 30ppm of the combination of extracts, the synthesized melanin concentration falls of 56% (*p <0.01 vs*. control). The decrease was 50% with 10 ppm (*p <0.01 vs*. control).

On these same cells, an evaluation of the expression of the TRP-1 protein (Tyrosinase Related Protein-1) was performed after immunofluorescent staining. This protein is one of the most important in the pigmentation process, since it directly modulates this process. The results demonstrate that 30ppm of the combination of extracts diminish the TRP-1 expression by 26% (*p<0.01, vs* negative control). These two results show the lightening feature of the combination of extracts.

Surprisingly, it is again highlighted for this activity a synergy between the three plants. Indeed, none of the extracts tested separately and under the same culture conditions on melanocytes present the effect of reducing the production of melanin.

### B) Galenics

### Active ingredient according to the invention:

Formula comprising the combination of extracts and in particular the active ingredient according to the invention recited in above example A).

Different formulations are described below, with or without additional cosmetic active ingredients, the latter coming in support and/or complement of the activity of the active ingredient according to the invention. These ingredients can be of any class according to their function(s), application site (body, face, neck, chest, hands, etc..), the final desired effect and the target consumer, for example anti-aging, anti-wrinkles, moisturizing, against dark circles, firming, lightening, anti-glycation, against spots, slimming, soothing, muscle relaxant, anti-redness, anti-stretch marks, etc..

### Example 1: Cream form

| | | **Day cream** | **Night cream** | **Lifting and anti-age cream** |
|---|---|---|---|---|
| **Ingredients** | **INCI name** | **Mass %** | **Mass %** | **Mass %** |
| **Phase A** | | | | |
| H₂O | Water | Qsp100 | Qsp100 | Qsp100 |
| Optasense G83™ | Carbomer | 0.30 | 0.30 | 0.30 |

| **Phase B** | | | | |
|---|---|---|---|---|
| Brij S2 SS™ | Steareth-2 | 0.40 | 0.40 | 0.40 |
| Brij S10 SO™ | Steareth-10 | 1.20 | 1.20 | 1.20 |
| Crodafos CES™ | Cetearyl alcohol & Dicetyl phosphate & Ceteth 10 phosphate | 4.0 | 4.00 | 4.00 |
| Crodacol CS 90™ | Cetearyl Alcohol | 1.50 | 1.50 | 1.50 |
| Laurocapram™ | Laurocapram | 2.50 | 2.50 | 2.50 |
| Cyclopentasiloxane (and) Cyclohexasiloxane | Cyclopentasiloxane (and) Cyclohexasiloxane | 2.00 | 2.00 | 2.00 |
| Crodamol OSU™ | Diethylhexyl succinate | 7.00 | 7.00 | 7.00 |

| **Phase C** | | | | |
|---|---|---|---|---|
| Potassium sorbate | Potassium Sorbate | 0.15 | 0.15 | 0.15 |

| **Phase D** | | | | |
|---|---|---|---|---|
| Glycerin | Glycerin | 2.00 | 2.00 | 2.00 |
| Phenoxyethanol | Phenoxyethanol | qs | qs | qs |

| **Phase E** | | | | |
|---|---|---|---|---|
| H₂O | Water | 4.00 | 4.00 | 4.00 |
| NaOH 30 % | Sodium Hydroxide | 0.40 | 0.40 | 0.40 |

| **Phase F** | | | | |
|---|---|---|---|---|
| Active ingredient according to Example A | | 3.00 | 4.00 | 3.00 |
| PRODIZIA™ | | | 3.00 | |
| IDEALIFT™ | | | | 4.00 |

**Protocol:** Weigh phase A and let swell without stirring for 30 minutes. Heat phase A at 75°C in a water bath. Weigh and mix phase B. Weigh phase C and heat to 75°C in a water bath, mix well. Add phase B to phase A. Mix well. Under stirring, pour phase C into phase A+B. Homogenise well. Add phase D, extemporaneously. Add phase E, mix well. Add phase F, homogenise well.

**Examples of other ingredients** that can be added to this cream type formulation in one of the phases or extemporaneously according to their hydrophobic or hydrophilic physical property, at a particular % according to their concentration and the desired effect:
DERMAXYL™: anti-aging active ingredient marketed by SEDERMA (WO2004/101609) which smoothes wrinkles and repair the skin barrier. 3 wt% of this ingredient may be for example added extemporaneously to phase (A+B+C).

Niacinamide (B3 vitamin), Retinol, Resveratrol, DHEA: anti-aging actives, including anti-wrinkle. 0.5% by weight of Retinol, Resveratrol or DHEA may be for example extemporaneously added in phase (A+B+C). 10 wt% of Niacinamide 10% in water can for example be added to the phase F.

Hexamidine: antibacterial active that may be added to the phase F of the formulation at 0.5% by weight.

CHRONODYN™: active ingredient marketed by SEDERMA (WO2006/075311), which tones and firms the skin, erases signs of fatigue. 3% of this ingredient can for example be added to the phase F.

VENUCEANE™: active ingredient marketed by SEDERMA (WO2002/066668) that prevents visible signs of photoaging (spots, wrinkles, dryness ...), protects cell structures caused by UV damage and enhances skin integrity. 3% of this ingredient can for example be added to the phase F.

IDEALIFT™: active ingredient marketed by SEDERMA (WO2010/136965 - Butylene Glycol (and) Water (and) Sorbitan Laurate (and) Hydroxyethylcellulose (and) Acetyl Dipeptide-1 Cetyl Ester) who fights flaccidity of the face and improves resistance to gravity. 4% of this ingredient can for example be added to the phase F.

PRODIZIA™ : active ingredient marketed by SEDERMA fighting the signs cutaneous fatigue caused by glycation and glycoxidation. 3% of this ingredient can for example be added to the phase F.

### Example 2: Balm form

| **Ingredients** | **INCI names** | **Mass %** |
|---|---|---|
| **Phase A** | | |
| H₂O | Water | qsp100 |
| Sodium benzoate | Sodium Benzoate | qs |
| Potassium sorbate | Potassium Sorbate | qs |
| Glycerin | Glycerin | 3.70 |
| Magnesium sulfate | Magnesium Sulfate | 0.70 |

| **Phase B** | | |
|---|---|---|
| Unsaponifiable shea butter | Shea Butter | 0.50 |
| Arlacel 986™ | Sorbitan Isostearate & Hydrogenated Castor Oil & Cera Alba & Stearic Acid | 9.00 |
| Syncrowax HRC™ | Tribehenin | 2.00 |
| Magnesium stearate | Magnesium Stearate | 1.00 |
| Pripure 3759™ | Squalane | 10.00 |
| Sweet almond oil | Prunus Amygdalus Dulcis Oil | 4.00 |
| Crodamol GTCC™ | Caprylic/Capric Triglyceride | 5.00 |
| Covi-Ox T90™ | Tocopherol | 0.10 |
| Argan oil | Argania Spinosa Kemel Oil | 1.00 |

| **Phase C** | | |
|---|---|---|
| Active ingredient according to the invention | | 3.00 |

| **Phase D** | | |
|---|---|---|
| Fragrance | Fragrance | 0.20 |

**Protocol:** Weigh phase A and put to heat in a water bath at 85°C. Weigh phase B and put to heat in a water bath at 85°C. Weigh phase C. Prior to forming the emulsion, add phase C to phase A, mix well. Slowly pour phase A+C in phase B with vigorous stirring. Mix thoroughly under stirring to cool. Add phase D in the emulsion around 35°C, mix well.

**Examples of other ingredients** that can be added to this formulation:
Covi-Ox T90™: antioxidant active (tocopherol) marketed by Cognis. It can be added to phase B, for example up to 0.1%.

Crodarom Goji™: anti-oxidant, moisturizing and detoxifying active marketed by Crodarom. It may be added simultaneously with phase C to phase A, for example up to 2.50%.

### Example 3: Gel form for face

| **Ingredients** | **INCI names** | **Mass** % |
|---|---|---|
| **Phase A** | | |
| H₂O | Water | Qsp100 |
| Cetyl hydroxyethylcellulose | Cetyl hydroxyethylcellulose | 0.30 |

| **Phase B** | | |
|---|---|---|
| Ultrez 10™ | Carbomer | 0.40 |
| H₂O | Water | 20.00 |

| **Phase C** | | |
|---|---|---|
| Glycerin | Glycerin | 3.00 |
| Panstat™ | Ethyl & Methyl & Propyl parabens | 0.30 |

| **Phase D** | | |
|---|---|---|
| Marcol 82™ | Mineral oil | 4.00 |
| Crillet 1™ | Polysorbate 20 | 1.00 |
| Crodamol AB™ | C12-15 Alkyl Benzoate | 2.00 |
| Pemulen TR2™ | C10-30 Alkyl Acrylate cross polymer | 0.30 |

| **Phase E** | | |
|---|---|---|
| Sorbate de potassium | Potassium Sorbate | 0.10 |

| **Phase F** | | |
|---|---|---|
| H₂O | Water | 5.00 |
| NaOH 10N | Sodium hydroxide | 0.50 |

| **Phase G** | | |
|---|---|---|
| Active ingredient according to the invention | | 2.00 |

**Protocol:** Disperse phase A under stirring. Sprinkle Ultrez 10 in water, let swell 30 minutes. Heat phase C until complete dissolution. Mix phase A with phase B. Add phase C in phase (B+A). Add phase D under stirring to phase (A+B+C). Add pPhase E. Neutralize with phase F. Add phase G and mix.

**Examples of other ingredients** that may be added to this gel-type formulation in one of the phases depending on their hydrophobic or hydrophilic physical property, at a certain% depending on their concentration and the desired effect:
RIGIN™: active marketed by SEDERMA (WO2000/433417) improving the elasticity and firmness of the skin, increasing hydration and smoothing the skin. 3% by weight of this ingredient may for example be added to the formulation in phase G.

RENOVAGE™: global anti-aging active ingredient marketed by SEDERMA (WO2006/020646). 3% by weight of this ingredient may for example be added to the formulation in phase D.

LUMISKIN™: active ingredient marketed by SEDERMA (WO2004/024695), which lightens the complexion. 3% by weight of this ingredient may for example be added to the formulation in phase D.

SUBLISKIN™: active ingredient marketed by SEDERMA (WO2009/055663) that moisturizes and smooths the skin while allowing it to resist to external aggressions. 3% by weight of the ingredient may for example be added to the formulation in phase G.

MATRIXYL™3000: peptide-based anti-wrinkle ingredient marketed by Sederma (WO2005/048968) comprising two matrikines Pal-GHK and Pal-GQPR, which in synergy helps repairing skin damages caused by aging. 3% by weight can be added for example in phase G of the formula.

MATRIXYL synthe'6™: peptide-based anti-wrinkle ingredient marketed by SEDERMA which helps repair skin damage caused by aging. 2% by mass of that ingredient may for example be added to the formulation in phase G.

### Example 4: Body cream form

| **Ingredients** | **INCI names** | **Mass** % |
|---|---|---|
| **Phase A** | | |
| H₂O | Water | qsp 100 |
| Ultrez 10™ | Carbomer | 0.40 |

| **Phase B** | | |
|---|---|---|
| Glycerin | Glycerin | 3.00 |
| Panstat ™ | Ethyl & Methyl & Propyl parabens | 0.30 |

| **Phase C** | | |
|---|---|---|
| Crill 3™ | Sorbitan Stearate | 2.00 |
| Marcol 82™ | Mineral oil | 4.00 |
| Cromollient DP3A™ | PPG 3 Myristyl Ether Adipate | 1.00 |
| Cithrol GMS AS™ | Glyceryl stearate & PEG 100 stearate | 3.00 |

| **Phase D** | | |
|---|---|---|
| Potassium sorbate | Sorbate de potassium | 0.10 |

| **Phase E** | | |
|---|---|---|
| NaOH 30% | Sodium hydroxide | 0.40 |
| H₂O | Water | 4.00 |

| **Phase F** | | |
|---|---|---|
| Active ingredient according to the invention | | 3.00 |

| **Phase G** | | |
|---|---|---|
| Fragrance | Fragrance | 0.10 |

**Protocol:** Weigh phase A and let swell for 30 minutes. Put phase A to heating in a water bath at 75°C. Heat phase B until dissolved. Add phase B to phase A. heat phase C in a water bath at 75°C. While stirring, add phase C in phase (A+B). Add phase D, mix well. Neutralize with phase I at around 55°C, mix well. Add phase F and phase G, mix well.

**Examples of other ingredients** that may be added to this cream-type formulation in one of the phases depending on their hydrophobic or hydrophilic physical property, at a certain% depending on their concentration and the desired effect:
JUVINITY™: active marketed by SEDERMA reducing signs of aging on the face and neckline, smoothing wrinkles, densifying and restructuring the dermis. 2% of this ingredient may for example be added extemporaneously to Phase (A+B+C).

Tocopherol or E vitamin: actives having anti-radical and anti-oxidant properties.

O.D.A. white™: active marketed by SEDERMA (WO1994/07837) which lightens the skin by reducing the synthesis of melanin. 1% of that ingredient may for example be added extemporaneously to phase (A+B+C).

Tocopherol (vitamin E) or α-lipoic acid (ALA): active with anti-oxidant and anti-radical properties. 0.5% by weight can be added for example to Phase (A+B+C).

Bio-Bustyl™: active marketed by SEDERMA based on peptides and a bacterial filtrate having a global action on firmness and tone of the bust. 3% of this ingredient can be added for example to phase G.

### Example 5: Serum form

| **Ingredients** | **INCI names** | **Mass** % |
|---|---|---|
| **Phase A** | | |
| Optasense G 40™ | Carbomer | 0.25 |
| H₂O | Water | Qsp100 |

| **Phase B** | | |
|---|---|---|
| Butylene Glycol | Butylene Glycol | 3.00 |
| Phenoxyethanol | Phenoxyethanol | 0.20 |

| **Phase C** | | |
|---|---|---|
| Crillet 1™ | Polysorbate 20 | 0.50 |
| DC 245™ | Cyclopentasiloxane | 1.00 |
| Crodamol CAP™ | Cetearyl Ethylhexanoate | 2.00 |
| Crodamol STS™ | PPG-3 Benzyl Ether Myristate | 0.50 |
| Pemulen TR2™ | Acrylates/C 10-30 Alkyl Acrylates cross polymer | 0.20 |

| **Phase D** | | |
|---|---|---|
| Potassium sorbate | Potassium Sorbate | 0,10 |

| **Phase E** | | |
|---|---|---|
| H₂O | Water | 4.00 |
| NaOH 30 % | Sodium Hydroxiide | 0.45 |

| **Phase F** | | |
|---|---|---|
| Active ingredient according to the invention | | 2.00 |

| **Phase G** | | |
|---|---|---|
| Fragrance | Fragrance | 0.10 |

**Protocol:** Phase A: sprinkle carbomer in water, let swell 15 minutes. Mix phase B. Pour phase B in phase A and homogenize. Then weigh phase C, mix and add in phase A+B under stirring. Let swell 1 hour. Extemporaneously add phase D in the previous phase under stirring. Neutralize with phase E. Put under stirring. Then add phase F. Allow mix at least 1 hour under stirring and then add phase G. Mix well.

**Examples of other ingredients** that may be added to this serum-type formulation in one of the phases depending on their hydrophobic or hydrophilic physical property, at a certain% depending on their concentration and the desired effect:
LUMISHERE™: active ingredient marketed by SEDERMA (WO2004/024695). It is the combination of diacétylboldine (DAB) encapsulated in polymethylmethacrylate microcapsules and titanium dioxide modified with manganese (TiO₂Mn). The TiO₂Mn gives the skin a unifying, mattifying and luminous effect and DAB provides a physiological lightening effect. 4% of this ingredient may for example be added to the Phase F of the formulation.

REVIDRATE™: active marketed by SEDERMA that in particular improves the cohesion of the epidermis and its hydration. 2% of this ingredient may for example be added to phase C of the formulation.

EVERMAT™: active marketed by SEDERMA (WO2007/029187), which decreases the secretion of sebum and thus participates in the treatment of oily skin. 4% of this ingredient may for example be added to phase F of the formulation.

HALOXYL™: active ingredient marketed by SEDERMA (WO2005/102266) for eye contour that resorbs the dark circles. 3% by weight of this ingredient may be added to phase E of the formulation.

WONDERLIGHT™: active marketed by SEDERMA (Caprylic / Capric Triglyceride (and) Humulus lupulus (Hops) Strobile), which helps reduce hyperpigmentation accentuated by age and stress. 3% of this ingredient dissolved in the Optasense G82 (Croda) for example, can be added in phase C and D of the formulation.

The addition of OSMOCIDE 4™, active marketed by SEDERMA (WO 97/05856 - Glycerin, Water (Aqua) (and) Caprylyl Glycol, Carbomer, Acrylates/C10-30 Alkyl Acrylate Crosspolymer), in phase D reduces the concentration of traditional conservatives.

### Example 6: Lotion form

| **Ingredients** | **INCI names** | **Mass %** |
|---|---|---|
| **Phase A** | | |
| H₂O | Water | Qsp100 |

| **Phase B** | | |
|---|---|---|
| Butylene Glycol | Butylene Glycol | 5.00 |
| Phenoxyethanol | Phenoxyethanol | 0.20 |

| **Phase C** | | |
|---|---|---|
| Crillet 1™ | Polysorbate 20 | 2.00 |
| Crodamol STS™ | PPG-3 Benzyl Ether Myristate | 0.10 |

| **Phase D** | | |
|---|---|---|
| Potassium sorbate | Potassium Sorbate | 0.10 |

| **Phase E** | | |
|---|---|---|
| Active ingredient according to the invention | | 2.00 |

| **Phase F** | | |
|---|---|---|
| Fragrance | Fragrance | 0.10 |

**Protocol:** Weigh phase A. Weigh phase B and mix. Add phase B into phase A under stirring for 30 minutes. Weigh phase C and homogenize until obtaining a homogeneous mixture. Add phase C into phase A+B under stirring. Add phase D in the previous phase. Add phase E still under stirring, mix well. Weigh phase F, stir and add to the previous phase, mix well.

**Examples of other ingredients** that may be added to this lotion-type formulation in one of the phases depending on their hydrophobic or hydrophilic physical property, at a certain% depending on their concentration and the desired effect:
EYELISS™: active ingredient marketed by Sederma (WO2003/068141) that helps prevent against the appearance of bags under the eyes. 3% of this ingredient can for example be added to formulation phase E.

Ac-Net™: an active sold by SEDERMA (WO2003/02828692) offering a complete treatment of oily and acne-prone skins. 3% of this ingredient may for example be added to Phase E of the formula.

EVERMAT™: mentionned above. 4% of this ingredient can be for example added to formulation phase E.

HYDRERGY™: active marketed by Sederma (WO2003/02828692) obtained by fermentation of a micro-algae Rhizobium melitoti, which is a long term moisturizing agent and which stimulates the synthesis of ATP. 3% may be added for example to phase E of the formula.

### Example 7: Mask form

| **Ingredients** | **INCI names** | **Mass %** |
|---|---|---|
| **Phase A** | | |
| H₂O | Water | Qsp100 |
| Sodium benzoate | Sodium benzoate | qs |
| Potassium sorbate | Potassium Sorbate | qs |

| **Phase B** | | |
|---|---|---|
| Zemea™ | Propandiol | 10.00 |
| Organic glycerin | Glycerin | 10.00 |
| Kelcogel CG HA™ | Gellan Gum | 0.50 |
| Jaguar S™ | Guar Gum | 0.60 |

| **Phase C** | | |
|---|---|---|
| Alcohol | Ethanol | 5.00 |

| **Phase D** | | |
|---|---|---|
| H₂O | Water | 1.00 |
| Lactic acid | Lactic acid | 0.10 |

| **Phase E** | | |
|---|---|---|
| Active ingredient according to the invention | | 3.00 |

| **Phase F** | | |
|---|---|---|
| Fragrance | Fragrance | 0.10 |

**Protocol:** Weigh phase A and put it under propeller stirring. Weigh and mix phase B. Slowly add phase B into phase A under propeller stirring. Heat phase A+B in a water bath at 80°C without stirring for 2 hours. Stir quickly, then let cool under stirring. Add phase C to the previous phase, at around 40°C. Adjust the pH to 5.50 with phase D, below 35°C, mix well. Add phase E, under slow stirring. Add phase F, mix well. Add phase G, mix well. Check that the pH is 5.0-5.5.

**Examples of other ingredients** that may be added to this formulation:
KOMBUCHKA™: active ingredient acting on complexion marketed by SEDERMA. 3.00% can be added at the end of the formulation before phase F.

### Example 8: Body fluid

| **Ingredients** | **INCI names** | **Mass %** |
|---|---|---|
| **Phase A** | | |
| H₂O | Water | 5.00 |
| Ultrez 10TM | Carbomer | 0.30 |

| **Phase B** | | |
|---|---|---|
| H₂O | Water | 74.30 |
| Natrosol 250™ | Hydroxyethyl cellulose | 0.20 |

| **Phase C** | | |
|---|---|---|
| Butylene Glycol | Butylene Glycol | 3.00 |
| Phenoxyethanol | Phenoxyethanol | 1.30 |

| **Phase D** | | |
|---|---|---|
| Crodamol AB™ | C12-15 Alkyl Benzoate | 2.00 |
| Crodamol GTCC™ | Caprylic/Capric Triglyceride | 3.00 |
| Crillet 1™ | Polysorbate 20 | 1.00 |
| Crodamol STS™ | PPG-3 Benzyl Ether Myristate | 1.00 |
| Pemulen TR2™ | C 10-30 Alkyl Acrylate Cross Polymer | 0.20 |

| **Phase E** | | |
|---|---|---|
| Sorbate | Potassium Sorbate | 0.10 |

| **Phase F** | | |
|---|---|---|
| NaOH 30 % | Sodium Hydroxyde | 0.50 |
| H₂O | Water | 5.00 |

| **Phase G** | | |
|---|---|---|
| Active ingredient according to the invention | | 3.00 |

| **Phase H** | | |
|---|---|---|
| Fragrance | Fragrance | 0.10 |

**Protocol:** phase A: sprinkle 10 Ultrez in water, let swell 30 minutes. Under stirring propeller v=300r/min sprinkle phase B and let rise for 1 hour. Add phase A to phase B under stirring propeller v=300r/min, mix well. Weigh and mix phase C. Weigh and mix phase D. Add phase C into phase A+B. Pour phase D in phase A+B+C. Mix well. Add Phase E and then phase F and then G, then add phase H. pHi = 6.2.

### Example 9: Slimming and anti-stretchmark cream

| **Ingredients** | **INCI names** | **Mass %** |
|---|---|---|
| **Phase A** | | |
| Demineralised water | Water (Aqua) | qsp 100% |
| Ultrez 10™ | Carbomer | 0.40 |

| **Phase B** | | |
|---|---|---|
| Glycerin | | 5.00 |
| Phenova™ | Phenoxyethanol (and) Mixed Parabens | 0.80 |

| **Phase C** | | |
|---|---|---|
| Crodamol OP™ | Ethylhexyl Palmitate | 4.00 |
| Crodacol CS90™ | Cetearyl alcoholCroda | 0.50 |
| Crodamol ML™ | Myristyl Lactate | 0.30 |
| Crillet 1™ | Polysorbate 20 | 1.00 |

| **Phase D** | | |
|---|---|---|
| Pemulen TR2™ | Acrylates / C10-30 Alkyl Acrylate Crosspolymer | 0.20 |
| DC 345™ | Cyclomethicone | 2.00 |

| **Phase E** | | |
|---|---|---|
| Potassium sorbate | Potassium Sorbate | 0.10 |

| **Phase F** | | |
|---|---|---|
| NaOH 38% | Sodium Hydroxide | 0.60 |
| Demineralised water | Water (Aqua) | 6.00 |

| **Phase G** | | |
|---|---|---|
| Active ingredient according to the invention | | 2.00 |

| **Phase H** | | |
|---|---|---|
| UNISLIM® (Sederma) | Ilex Paraguariensis (Leaf) Extract - Aqua (Water) - Butylene Glycol - Coffea Arabica (Coffee Seed) Bean Extract - PEG-60 Almond Glycerides - Glycerin - Cetyl Hydroxyethylcellulose | 3.00 |
| MATRIXYL® 3000 (Sederma) | Glycerin (and) Butylene Glycol (and) Aqua (Water) (and) Carbomer (and) Polysorbate-20 (and) Palmitoyl Oligopeptide (and) Palmitoyl Tetrapeptide-3 | 3.00 |
| DARUTOSIDE™ (Sederma) | Siegesbeckia Orientalis Extract | 3.00 |

**Protocol:** This emulsion is prepared as following: phase A: disperse Ultrez 10 in water and let swell 20 minutes. Mix phase B and heat at 60°C until dissolved. Add phase B to phase A under stirring. Heat phase (A+B). Weigh phase C and heat at 75°C. Add phase C to phase (A+B) under stirring. Mix thoroughly, then add phase D. Extemporaneously add phase E, then at about 50°C, neutralized with phase F. Add phases G and H at 35°C, pH ∼ 6.30.

### Example 10: Capilar lotion

| **Ingredients** | **INCI names** | **Mass** % |
|---|---|---|
| **Phase A** | | |
| Incroquat CTC 30™ | Cetrimonium chloride | 1.00 |
| Citric acid | Citric Acid | 0.22 |
| Trisodic citrate | Trisodic Citrate | 1.20 |
| Sorbate | Potassium Sorbate | 0,10 |
| H₂O | | Qsp |

| **Phase B** | | |
|---|---|---|
| Nipagine™ | Methyl paraben | 0.20 |
| Procetyl AWS™ | PPG 5 Ceteth 20 | 2.00 |

| **Phase C** | | |
|---|---|---|
| Active ingredient according to the invention | | 2.00 |

| **Phase D** | | |
|---|---|---|
| Crillet 1 ™ | Polysorbate 20 | 1.00 |
| Fragrance | Fragrance | 0.10 |

### Example 11: Moisturazing foundation

| **Ingredients** | **INCI names** | **Mass %** |
|---|---|---|
| **Phase A** | | |
| Demineralised water | Water (Aqua) | qsp 100% |
| KOH 10% | Potassium hydroxide | 1.30 |
| Crillet 4 NF™ | Polysorbate 80 | 0.10 |

| **Phase B** | | |
|---|---|---|
| Titanium dioxide | | 6.00 |
| Talc | | 3.05 |
| Yellow iron oxide | | 1.80 |
| Red iron oxide | | 1.00 |
| Black iron oxide | | 0.15 |

| **Phase C** | | |
|---|---|---|
| Propylene glycol | | 4.00 |
| Veegum Regular | Magnesium Aluminum Silicate | 1.00 |

| **Phase D** | | |
|---|---|---|
| Propylene glycol | | 2.00 |
| Cellulose gum | Sodium Carboxymethylcellulose | 0.12 |

| **Phase E** | | |
|---|---|---|
| Cromollient DP3-A™ | Di-PPG-3 Myristyl Ether Adipate | 12.00 |
| Crodamol ISNP™ | Isostearyl Neopentanoate | 4.00 |
| Crodafos CS 20™ | Cetearyl Alcohol (and) Ceteth-20 Phosphate (and) Dicetyl Phosphate | 3.00 |
| Volpo S-10™ | Steareth-10 | 2.00 |
| Crodacol C-70™ | Cetyl alcohol | 0.62 |
| Volpo S-2™ | Steareth-2 | 0.50 |

| **Phase F** | | |
|---|---|---|
| Moist24 ® | Imperata Cylindrica extract (and) water (and) glycerine (and) PEG8 (and) carbomer | 3.00 |

| **Phase G** | | |
|---|---|---|
| Active ingredient according to the invention | | 5.00 |

| **Phase H** | | |
|---|---|---|
| Germaben II | Propylene glycol (and) Diazolidinyl Urea (and) Methylparaben (and) Propylparaben | 1.00 |

**Protocol:** Phase A: disperse Ultrez 10 in water and let swell 20 minutes. Premix pigments, then add phase B to phase A until the pigments are dispersed, start heating. Heat phase C separately, mix well. Pour phase C in phase (A+B) under stirring. Prepare phase D separately, mix well. Pour phase D in phase (A+B+C) under stirring, homogenize. Prepare phase E separately, mix well. Pour in phase E in phase (A+B+C+D) under stirring, around 45°C add phase F and then G and H. Adjust the pH to 7.5, homogenize.

Moist 24®: extract of *Cylindrica imperata* roots marketed by SEDERMA (WO 01/62218)

### Example 12: Lip balm

| **Ingredients** | **INCI names** | **Mass %** |
|---|---|---|
| **Phase A** | | |
| Demineralised water | qsp | qsp 100% |
| Sorbate | Potassium Sorbate | 0.10 |
| Magnesium sulfate | Magnesium Sulfate | 0.70 |

| **Phase B** | | |
|---|---|---|
| Abil EM 90™ | Cetyl Dimethicone Copolyol | 3.00 |
| Methyl paraben | Methyl Paraben | 1.00 |
| Syncrowax HRC™ | Tribehenin | 0.30 |
| Crodamol STS™ | PPG-3 Benzyl Ether Myristate | 2.00 |
| Mineral Oil | Mineral Oil | 19.00 |

| **Phase C** | | |
|---|---|---|
| Active ingredient according to the invention | | 1.00 |

| **Part D** | | |
|---|---|---|
| Fragrance | Fragrance | 0.10 |

**Protocol:** Heat phase A at 85°C. Mix phase B and heat at 85°C. Mix. Pour slowly phase A into phase B under stirring (Staro s = 3000, then s = 1200). Add phase C preheated at 80°C, homogenize. Add phase D around 35°C. Cast.

### Example 13: Protective capilar spray

| **Ingredients** | **INCI names** | **Mass %** |
|---|---|---|
| **Phase A** | | |
| Demineralised water | Water (Aqua) | qsp 100% |
| Ethanol | | 10.00 |
| Crillet 1™ | Polysorbate 20 | 0.40 |
| Incroquat CTC 30™ | Cetrimonium Chloride | 1.00 |

| **Phase B** | | |
|---|---|---|
| HELIOGENOL™ | Butylene Glycol (and) Helianthus Annuus (Sunflower) Seed Extract | 5.00 |
| Conservatives | | qs |

| **Phase C** | | |
|---|---|---|
| Active ingredient according to the invention | | 4.00 |

| **Phase D** | | |
|---|---|---|
| Demineralised water | Water (Aqua) | 0.50 |
| NaOH | Sodium Hydroxide | 0.05 |

**Protocol:** Weigh and mix phase A under stirring (propeller v = 300 r/ min); Add phase B, homogenize, then phase C. Adjust the pH to ∼ 5-5.5 with phase D.

HELIOGENOL™ is a sunflower seed extract proposed by Sederma (EP0512040).

### C) In vivo studies

The activity of the active ingredient of the invention on the improvement of the general condition of the skin, mucous and appendages and in particular on signs of aging or imperfections has been shown in *in vivo* tests given below.

A day cream prepared according to Example 1 was used for these tests. The placebo was prepared according to the same example, by replacing the active ingredient according to the invention by the excipient ingredient.

### Principle

The study of the efficacy was conducted on a panel of 23 women, of whom 2/3 were Chinese or of other Asian descent. These women, who were 40 to 75 years of age (mean age 57.5), were chosen for their visible signs of ageing (wrinkles and fine lines) associated with irregular complexion with pigmentation defaults such as hyperpigmented spots and areas.

Several complementary, non-invasive methods were combined for this study:
- Transepidermal water loss (TEWL) was measured using a Vapometer® to determine the skin barrier's resistance.
- The clarity and uniformity of the skin was measured through the analysis of standardized photo images.
- Melanin content of pigmentary defaults was measured using a Mexameter®.
- Expert judges clinically evaluated five signs of ageing by using standardised photos.

### Protocol

### Specific inclusion criteria

Volunteers were asked to observe a 15-day wash-out period in which they refrained from using any cosmetic products. They were also asked to maintain hormonal constancy (i.e., refrain from changing contraceptive, substitute or curative hormonal treatments) for the three months prior to the test as well as during the test. Volunteers were required to use only the cosmetics provided for the duration of the study.

### Type of studies and duration

This was a single-blind study of the face and forearms (day cream with 3% of the active ingredient according to the invention and placebo cream were applied contralaterally). The creams were massaged into the skin twice daily for 2 months.

The study synopsis can be summarised according to the diagram below.

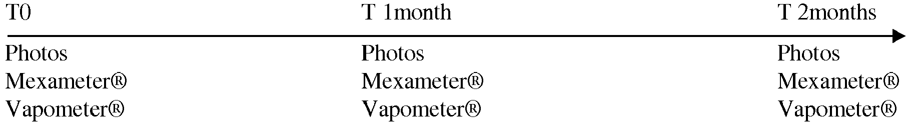

The statistical studies were performed using the Student's *t*-test or the non-parametric Wilcoxon signed-rank test when necessary. In both cases, the unilateral tests were performed on matched series.

### 1. Measurement of the resistance of the skin barrier by quantifying TEWL using the Vapometer®

The skin is the body's first line of defense. As a result, its ability to protect the body from aggressive external factors is extensive. A decrease in this resistance leads to imbalances and creates disorders that make the skin more sensitive and less uniform.

This ability to resist was assessed by measuring the effects of a slight disruption in the skin barrier caused by repeated stripping. At T0, the basal transepidermal water loss (TEWL) of the forearm was measured using the Vapometer® (Delfin). A series of controlled strippings was then performed to slightly modify the homeostasis of the barrier. Several dozens of minutes later, the increase in TEWL caused by the stripping was measured under stabilised conditions.

After one or two months of applying the cream according to the invention or the placebo cream, a protocol identical to that at T0 was employed. A decrease in the TEWL elevation indicated improved resistance.

**Table 3:**

| | **TEWL elevation (in g/m²/h)** | | |
|---|---|---|---|
| | **T0** | **T 1 month** | **T 2 months** |
| **Cream according to the invention** | 4.33 ± 2.78 | 3.71 ± 2.05 | 2.78 ± 1.82 |
| Placebo | 4.24 ± 2.97 | 4.86 ± 2.66 | 3.66 ± 1.89 |
| **Delta vs placebo** | +0.09 | -1.15 | -0.88 |
| **% change vs placebo** | **+2%** | **-23.7%** | **-24%** |
| **Significance** | *nsd* | *p<0.01* | *p<0.05* |

The results demonstrate that the rupture in the barrier created at T0 caused an identical rise in TEWL at the two sites to receive the cream according to the invention or the placebo cream (2% difference, *nsd*).

After 1 month of the invention cream or placebo cream application, the same stripping procedure as at T0 demonstrated a clear difference, with an improvement at the invention site (-23.7% *vs* placebo, *p<0.01).* The loss in homeostasis related to the stripping was markedly reduced on the invention side: +4.33 units to +3.71 units.

The rupture in homeostasis was even less marked after two months of invention cream application (only 2.78 units). Compared with placebo, the observed loss in homeostasis was still 24% less pronounced (*p<0.05*).

### 2. Measurement of the clarity (radiance) and uniformity of skin by analysing standardised photos

To perform these measurements, standardised photos were taken using a photographic bench (commercialized by Orion Concept) composed of a high definition digital camera, special lighting and a system for keeping volunteers in place.

The position of the volunteers, the photographic parameters and the lighting were thus standardised and controlled to be reproducible over time. Furthermore, a colorimetric scale was used to perform colour and spatial calibration between each take to ensure perfect standardisation.

### 2.1. Clarity of hyperpigmented areas

The clarity of the skin was quantified using photographs taken in crossed polarised light to remove any shine. A hyperpigmented area was selected on each face and the L* (clarity) parameter was analysed using FrameScan™ (Orion Concept) software. The L* parameter of the CIELab system represents the colorimetric component and varies from black (L*=0) to white (L*=100); an increase in L*, representing a lightening of the hyperpigmented area, was sought. Since the clarity of a skin does not change from one extreme of the scale to the other, however, a constant value of L*=35 was selected to provide a more physiological scale.

**Table 4: Variation of the clarity (L*) of the hyperpigmented areas (n=22 volonteers)**

| | **Cream according to the invention** | | | **Placebo** | | |
|---|---|---|---|---|---|---|
| | **T0** | **T 1month** | **T 2months** | **T0** | **T 1month** | **T 2months** |
| **Mean** | 12.12 | 13.24 | 12.58 | 12.24 | 12.83 | 12.06 |
| **± SD** | ± 4.64 | ± 4.82 | ± 4.89 | ± 5.36 | ± 4.99 | ± 5.49 |
| **% variation *vs*.T0** | | **9.3%** | **3.84%** | | 4.84% | -1.45% |
| **Maximum** | | 37% | 34% | | | |
| **% vs placebo** | | **4.46%** | **5.29%** | | | |

These results demonstrate that the application of the cream according to the invention leads to, in the first month, a significant lightening of the hyperpigmented areas with a 4.5% improvement compared with the placebo-treated site. The effect is sustained at T2 months with a 5.3% increase in lightening compared with placebo. The decrease with respect to L* at T2 months is explained by the increase in sunlight (month of May). However, the product/placebo difference still remained.

### 2.2. Complexion uniformity

As for clarity, photos taken in crossed polarised light were used to assess complexion uniformity. A vast area corresponding to the cheek was selected and the area's texture features were analysed using Colorskin (Newtone) software. Texture features are traditionally used in aerial or satellite imagery to characterise and classify the different terrains. More recently, they were used on skin to identify melanomas and characterise ageing or hyperpigmentation. This uniformity parameter was used to demonstrate an overall improvement in complexion.

**Table 5: Variation in complexion uniformity (arbitrary unit x10⁵; n=22 volonteers)**

| | **Cream according to the invention** | | | **Placebo** | | |
|---|---|---|---|---|---|---|
| | **T0** | **T 1month** | **T 2months** | **T0** | **T 1month** | **T 2months** |
| **Mean ± SD** | 447 ± 126 | 468 ± 148 | 480 ± 160 | 460 ± 149 | 457 ± 183 | 459 ± 158 |
| **% variation *vs*. T0** | | **4.7%** | **7.4%** | | -0.6% | -0.2% |
| **Significance** | | *p*=*0.05* | *p<0.05* | | *nsd* | *nsd* |
| **Maximum** | | 33% | 31% | | | |
| **Responders** | | 64% | 77% | | | |
| **% vs placebo** | | **+5.3%** | **+7.6%** | | | |
| **Significance** | | *p=0.15* | *p<0.05* | | | |

There was considerable complexion uniformity observed after 1 month of the application of the cream of the invention, since a +4.7% increase (*p*=*0.05*) is to be compared with the slight decrease of 0.6% observed on the placebo side. This trend was confirmed after 2 months of the invention cream application, with a significant improvement in uniformity of +7.4% (*p<0.05*) while the placebo effect was null (-0.2%).

### 3. Evaluation of melanin content using a Mexameter®

The Mexameter MX18® (Courage & Khazaka) is generally used in cosmetic evaluations due to its reliability and its correlation with other machines. It was used to quantify skin coloration flaws thanks to its 5-mm probe, which enables precise, targeted measurements of pigmented spots. The probe emits rays of 3 different wavelengths (568 nm=green, 660 nm=red, 880 nm=infrared), the latter two providing the index of melanin.

In this test, the measurement of a non-pigmented area was taken from the measurement obtained on the adjacent hyperpigmented area, which enables variations due to interference, such as from sunlight, to be smoothed.

**Table 6: Variation in the quantity of melanin (Mexameter pigmented area index - control index, 21 volonteers, n=3)**

| | **Cream according to the invention** | | | **Placebo** | | |
|---|---|---|---|---|---|---|
| | **T0** | **T 1month** | **T 2months** | **T0** | **T 1month** | **T 2months** |
| **Mean ± SD** | 81.4 ± 40.2 | 70.9 ± 36.1 | 65.9 ± 36.7 | 75.4 ± 33.4 | 76.4 ± 38.5 | 74.8 ± 35.3 |
| **% variation *vs*. T0** | | **-12.8%** | **-19%** | | 1.3% | -0.8% |
| **Significance** | | *p<0.01* | *p<0.01* | | *nsd* | *nsd* |
| **Maximum** | | -54% | -60% | | | |
| **Responders** | | 86% | 86% | | | |
| **Variation % vs placebo** | | **-14.1%** | **-18.2%** | | | |
| **Significance** | | *p<0.01* | *p<0.01* | | | |

These results complete those already obtained. They demonstrate the benefits of the cream according to the invention in reducing complexion inhomogeneity measured here on the pigmented area. After one month of application, the cream of the invention reduces melanin content in hyperpigmented areas by 14.1% (*p<0.01 vs* placebo), a phenomenon that was even greater at two months (-18.2% *vs* placebo, *p<0.01).*

In support of these results, in corneocytes sampled using adhesives and colored using the Fontana Masson method, there were fewer observed melanin inclusions on the side that received the cream according to the invention than on the placebo side.

### 4. Clinical evaluation of five signs of ageing

The five signs of ageing taken into consideration were:
- Facial fine lines and wrinkles
- Lip lines and wrinkles
- Pigment spot density
- Spot size
- Pigmentation intensity

This evaluation of the five signs was performed by four expert judges using photographs that were taken previously (cf § 2) in diffused light for an appearance that was as close as possible to reality. The evaluation was performed under perfectly standardised conditions based on a scale of 0 to 7. A photo scale was generally used. A graph was obtained.

Moreover, the five signs were also averaged to provide a global ageing severity index. A decrease in rating according to this index indicated an improvement.

**Table 7: Global ageing severity index (Mean of 5 clinical signs, 22 volunteers, n=4 judges)**

| | **Cream according to the invention** | | **Placebo** | |
|---|---|---|---|---|
| | **T0** | **T 2months** | **T0** | **T 2months** |
| **Mean ± SD** | 2.79 ± 1.01 | 2.56 ± 1.00 | 2.64 ± 0.97 | 2.57 ± 0.99 |
| **% variation *vs*. T0** | | **-8.2%** | | -2.7% |
| **Significance** | | *p<0.01* | | *nsd* |
| **Maximum** | | -0.73 (-20%) | | |
| **Responders** | | 82% | | |
| Significance *vs*. placebo | | *p<0.01* | | |

The results demonstrate a clear improvement in the five indices after applying the cream of the invention. Depending on the parameter under consideration, the individual variations ranged from -1% to -13%, and the most intense decreases were observed for facial wrinkles and fine lines, but also for pigment spot intensity and pigment spot density.

Furthermore, the global ageing severity index demonstrates a significant -8.2% decrease. Compared with the placebo, the observed decrease was significant (p<0.01) since with the placebo the index was only slightly lower (-2.7%) compared with T0.

## Claims

1. Use of a cosmetic active ingredient comprising (i) an extract of *Astragalus membranaceus,* (ii) an extract of *Bupleurum falcatum,* and (iii) an extract of *Atractylodes macrocephalae* or of a composition comprising said ingredient in a physiological acceptable medium, in a cosmetic, non-therapeutical, topical treatment for improving the appearance and general condition of the skin and its annexes, in particular for treating and/or preventing ageing signs and/or imperfections of the skin and its annexes.

2. Use according to claim 1, wherein the topical cosmetic treatment is a treatment of wrinkles and fine lines.

3. Use according to claim 1 or 2, wherein the topical cosmetic treatment is a treatment for reducing the complexion inhomogeneity.

4. Use according to anyone of claims 1 to 3, wherein the topical cosmetic treatment is a treatment for improving skin radiance.

5. Use according to anyone of claims 1 to 4, wherein the topical cosmetic treatment is an anti-oxidant treatment.

6. Use according to anyone of claims 1 to 5, wherein the topical cosmetic treatment is a treatment of skin dehydration.

7. Use according to anyone of claims 1 to 6, wherein the topical cosmetic treatment is for improving the mechanical properties of the skin, comprising firmness and elasticity.

8. Use according to anyone of claims 1 to 7, wherein the topical cosmetic treatment is for improving the skin barrier function.

9. Use according to anyone of claims 1 to 8, wherein the topical cosmetic treatment is for improving the dermo-epidermal junction.

10. Use according to anyone of claims 1 to 8, wherein the ingredient consists of (i) an extract of *Astragalus membranaceus,* (ii) an extract of *Bupleurum falcatum,* and (iii) an extract of *Atractylodes macrocephalae.*

11. Use according to anyone of claims 1 to 10, wherein the composition comprises an additional active ingredient.

12. Use according to claim 11, wherein said additional active ingredient is chosen among lightening, anti-redness, anti-spots, calming, for the treatment of sensitive, reactive skins, UV sunscreens, moisturizing, humectants, exfoliants, smoothing, toning, anti-aging, anti-wrinkles, slimming, volumizing, improving the mechanical and elastic properties, radiance, detoxifying, acting on the skin barrier, anti-acne, affecting the secretion of sebum, matting, unifying, anti-oxidants, anti-radical, anti-glycation, propigmenting or depigmentation, depilatories, anti-hair regrowth, or promoting hairs and body hair growth, eye contours (dark circles and under eye bags), promoting blood circulation, peptides and vitamin agents.

13. Use according to anyone of claims 1 to 12, wherein the skin is Asian.

14. Cosmetic active ingredient consisting of (i) an extract of *Astragalus membranaceus,* (ii) an extract of *Bupleurum falcatum,* and (iii) an extract of *Atractylodes macrocephalae.*

15. Ingredient according to claim 14, wherein said extracts are obtained from roots or rhizomes of the plants.

16. Woven or non-woven material comprising the cosmetic active ingredient according to claim 14 or 15.

## Patentansprüche

1. Verwendung eines kosmetischen Wirkstoffs, umfassend (i) einen Extrakt von *Astragalus membranaceus,* (ii) einen Extrakt von *Bupleurum falcatum* und (iii) einen Extrakt von *Atractylodes macrocephalae* oder eine Zusammensetzung, die den Bestandteil in einem physiologisch verträglichen Medium enthält, in einer kosmetischen, nicht-therapeutischen, topischen Behandlung zur Verbesserung des Aussehens und des allgemeinen Zustands der Haut und ihrer Hautanhangsgebilde, insbesondere zur Behandlung und/oder Vorbeugung von Alterungsanzeichen und/oder Unvollkommenheiten der Haut und ihrer Hautanhangsgebilde.

2. Verwendung nach Anspruch 1, wobei die topische kosmetische Behandlung eine Behandlung von Falten und Fältchen ist.

3. Verwendung nach Anspruch 1 oder 2, wobei die topische kosmetische Behandlung eine Behandlung zur Verringerung der Teint-Ungleichmäßigkeit ist.

4. Verwendung nach einem der Ansprüche 1 bis 3, wobei die topische kosmetische Behandlung eine Behandlung zur Verbesserung des Hautglanzes ist.

5. Verwendung nach einem der Ansprüche 1 bis 4, wobei die topische kosmetische Behandlung eine antioxidative Behandlung ist.

6. Verwendung nach einem der Ansprüche 1 bis 5, wobei die topische kosmetische Behandlung eine Behandlung der Hautdehydrierung ist.

7. Verwendung nach einem der Ansprüche 1 bis 6, wobei die topische kosmetische Behandlung zur Verbesserung der mechanischen Eigenschaften der Haut, umfassend Festigkeit und Elastizität, dient.

8. Verwendung nach einem der Ansprüche 1 bis 7, wobei die topische kosmetische Behandlung zur Verbesserung der Hautbarrierefunktion dient.

9. Verwendung nach einem der Ansprüche 1 bis 8, wobei die topische kosmetische Behandlung zur Verbesserung der dermo-epidermalen Verbindung dient.

10. Verwendung nach einem der Ansprüche 1 bis 8, wobei der Bestandteil aus (i) einem Extrakt von *Astragalus membranaceus,* (ii) einem Extrakt von *Bupleurum falcatum* und (iii) einem Extrakt von *Atractylodes macrocephalae* besteht.

11. Verwendung nach einem der Ansprüche 1 bis 10, wobei die Zusammensetzung einen zusätzlichen Wirkstoff umfasst.

12. Verwendung nach Anspruch 11, wobei der zusätzliche Wirkstoff ausgewählt ist unter Aufhellung, Anti-Rötung, Anti-Flecken, beruhigend, zur Behandlung von empfindlicher, reaktiver Haut, UV-Lichtschutzmitteln, feuchtigkeitsspendenden Mitteln, Feuchthaltemitteln, Peelings, Glättung, Tonisierung, Anti-Aging, Anti-Falten, verschlankend, volumenspendend, Verbesserung der mechanischen und elastischen Eigenschaften, Glanz, entgiftend, auf die Hautbarriere wirkend, Anti-Akne, die Sekretion von Talg beeinflussend, mattierend, vereinheitlichend, Antioxidantien, Anti-Radikal-Mittel, Anti-Glykations-Mittel, Pigmentierung oder Depigmentierung, Enthaarungsmitteln, Anti-Haarwuchs oder Wachstumsförderung von Haar und Körperbehaarung, Augenkonturen (dunkle Ringe und Tränensäcke), Förderung der Blutzirkulation, Peptide und Vitamin-Agenzien.

13. Verwendung nach einem der Ansprüche 1 bis 12, wobei die Haut asiatisch ist.

14. Kosmetischer Wirkstoff, bestehend aus (i) einem Extrakt von *Astragalus membranaceus,* (ii) einem Extrakt von *Bupleurum falcatum* und (iii) einem Extrakt von *Atractylodes macrocephalae.*

15. Bestandteil nach Anspruch 14, wobei die Extrakte aus Wurzeln oder Rhizomen der Pflanzen erhalten werden.

16. Gewebe oder Vliesstoff, umfassend den kosmetischen Wirkstoff nach Anspruch 14 oder 15.

## Revendications

1. Utilisation d'un ingrédient actif cosmétique comprenant (i) un extrait d'*Astragalus membranaceus,* (ii) un extrait de *Bupleurum falcatum,* et (iii) un extrait d'*Atractylodes macrocephalae* ou d'une composition comprenant ledit ingrédient dans un milieu physiologique acceptable, dans un traitement cosmétique non-thérapeutique topique pour améliorer l'apparence et l'état général de la peau et de ses annexes, en particulier pour traiter et/ou prévenir les signes du vieillissement et/ou les imperfections de la peau et de ses annexes.

2. Utilisation selon la revendication 1, dans laquelle le traitement cosmétique topique est un traitement des rides et des ridules.

3. Utilisation selon la revendication 1 ou 2, dans laquelle le traitement cosmétique topique est un traitement pour réduire l'inhomogénéité du teint de la peau.

4. Utilisation selon l'une quelconque des revendications 1 à 3, dans laquelle le traitement cosmétique topique est un traitement pour améliorer l'éclat du teint de la peau.

5. Utilisation selon l'une quelconque des revendications 1 à 4, dans laquelle le traitement cosmétique topique est un traitement anti-oxydant.

6. Utilisation selon l'une quelconque des revendications 1 à 5, dans laquelle le traitement cosmétique topique est un traitement de la déshydratation de la peau.

7. Utilisation selon l'une quelconque des revendications 1 à 6, dans laquelle le traitement cosmétique topique est un traitement pour améliorer les propriétés mécaniques de la peau, comprenant la fermeté et l'élasticité.

8. Utilisation selon l'une quelconque des revendications 1 à 7, dans laquelle le traitement cosmétique topique est un traitement pour améliorer la fonction barrière de la peau.

9. Utilisation selon l'une quelconque des revendications 1 à 8, dans laquelle le traitement cosmétique topique est un traitement pour améliorer la jonction derme-épiderme.

10. Utilisation selon l'une quelconque des revendications 1 à 8, dans laquelle l'ingrédient consiste en (i) un extrait d'*Astragalus membranaceus,* (ii) un extrait de *Bupleurum falcatum,* et (iii) un extrait d'*Atractylodes macrocephalae.*

11. Utilisation selon l'une quelconque des revendications 1 à 10, dans laquelle la composition comprend un ingrédient actif additionnel.

12. Utilisation selon la revendication 11, dans laquelle ledit ingrédient actif additionnel est choisi parmi les actifs éclaircissants, anti-rougeurs, anti-taches, calmants, pour le traitement des peaux sensibles, réactives, les filtres solaires UV, les actifs hydratants, humectants, exfoliants, lissants, tonifiants, anti-âges, anti-rides, amincissants, volumateurs, améliorant les propriétés mécaniques et élastiques, l'éclat du teint, les actifs détoxyfiants, agissant sur la barrière cutanée, anti-acnés, agissant sur la sécrétion de sébum, matifiants, unifiants, anti-oxydants, anti-radicalaires, anti-glycants, propigmentants ou dépigmentants, dépilatoires, anti-repousse poils, ou favorisant la pousse des poils et des cheveux, contours des yeux (anti-cemes et anti-poches), favorisant la circulation sanguine, peptides et vitamines.

13. Utilisation selon l'une quelconque des revendications 1 à 12, dans laquelle la peau est une peau asiatique.

14. Ingrédient actif cosmétique constitué (i) d'un extrait d'*Astragalus membranaceus,* (ii) d'un extrait de *Bupleurum falcatum,* et (iii) d'un extrait d'*Atractylodes macrocephalae.*

15. Ingrédient selon la revendication 14, dans lequel lesdits extraits sont obtenus à partir de racines ou de rhizomes des plantes.

16. Matériau tissé ou non-tissé comprenant l'ingrédient actif cosmétique selon la revendication 14 ou 15.


SEQUENCE LISTING

<110>  SEDERMA

<120>  COMBINATION OF PLANT EXTRACTS, COSMETIC INGREDIENT AND
       COMPOSITION CONTAINTING IT, AND TOPICAL COSMETIC USE THEREOF

<130>  TCM PCT

<150>  FR1260377
<151>  2012-10-30

<160>  10

<170>  PatentIn version 3.5

<210>  1
<211>  6
<212>  PRT
<213>  artificial sequence

<220>
<223>  synthetic peptide


<220>
<221>  MOD_RES
<222>  (1)..(1)
<223>  amidation by a Palmitoyl chain on the N terminal end

<400>  1

Val Gly Val Ala Pro Gly
1               5


<210>  2
<211>  5
<212>  PRT
<213>  artificial sequence

<220>
<223>  synthetic peptide


<220>
<221>  MOD_RES
<222>  (1)..(1)
<223>  amidation by a Palmitoyl chain on the N terminal end

<400>  2

Lys Thr Thr Lys Ser
1               5


<210>  3
<211>  4
<212>  PRT
<213>  artificial sequence

<220>
<223>  synthetic peptide

<220>
<221>  MOD_RES
<222>  (1)..(1)
<223>  amidation by a Palmitoyl chain on the N terminal end

<400>  3

Gly Gln Pro Arg
1


<210>  4
<211>  5
<212>  PRT
<213>  artificial sequence

<220>
<223>  synthetic peptide

<400>  4

Lys Thr Thr Lys Ser
1               5


<210>  5
<211>  4
<212>  PRT
<213>  artificial sequence

<220>
<223>  synthetic peptide

<400>  5

Arg Ser Arg Lys
1


<210>  6
<211>  4
<212>  PRT
<213>  artificial sequence

<220>
<223>  synthetic peptide

<400>  6

Gly Gln Pro Arg
1


<210>  7
<211>  4
<212>  PRT
<213>  artificial sequence

<220>
<223>  synthetic peptide

<400>  7

Lys Thr Phe Lys
1



<210>  8
<211>  6
<212>  PRT
<213>  artificial sequence

<220>
<223>  Synthetic peptide

<400>  8

Gly Lys Thr Thr Lys Ser
1               5



<210>  9
<211>  6
<212>  PRT
<213>  artificial sequence

<220>
<223>  synthetic peptide

<400>  9

Val Gly Val Ala Pro Gly
1               5



<210>  10
<211>  5
<212>  PRT
<213>  artificial sequence

<220>
<223>  synthetic peptide



<220>
<221>  MOD_RES
<222>  (1)..(1)
<223>  amidation by a Palmitoyl chain on the N terminal end

<220>
<221>  MISC_FEATURE
<222>  (5)..(5)

<220>
<221>  MISC_FEATURE
<222>  (5)..(5)
<223>  Xaa being either a Methionine M or a Leucine L.

<400>  10

Tyr Gly Gly Phe Xaa
1               5